# EUROPEAN PATENT SPECIFICATION

(11) **EP 1 606 307 B1**
(45) Date of publication and mention of the grant of the patent: **24.10.2007**
(21) Application number: 03763151.2
(22) Date of filing: 02.07.2003
(51) Int. Cl.: C07J 71/00

(54) **ESTRIENO[3,2-B]/[3,4-C]PYRROLE DERIVATIVES USEFUL AS MODULATORS OF THE ESTROGEN RECEPTORS**
ESTRIENO[3,2-B]/[3,4-C]PYRROLE DERIVATIVE ALS MODULATOREN DES ÖSTROGEN REZEPTORS
DERIVES D'ESTRIENO[3,2-B]/[3,4-C]PYRROLE COMME MODULATEURS DE RECEPTEUR D'OESTROGENES

(30) Priority: 03.07.2002 US 393608 P
(43) Date of publication of application: 21.12.2005
(73) Proprietor: Ortho-McNeil Pharmaceutical, Inc., Raritan, NJ 08869-0602 (US)
(72) Inventor: SUI, Zhihua Johnson & Johnson PRD, Raritan, NJ 08869-0602 (US); ZHANG, Xuqing Johnson & Johnson PRD, Raritan, NJ 08869-0602 (US)
(74) Representative: Mercer, Christopher Paul
(86) International application number: PCT/US2003/020935
(87) International publication number: WO 2004/005314

(56) References cited:
- US-A- 3 484 435
- ZHANG X ET AL: "An efficient synthesis of novel estrieno[2.3-b] and [3.4-c]pyrroles" TETRAHEDRON LETTERS, ELSEVIER SCIENCE PUBLISHERS, AMSTERDAM, NL, vol. 44, no. 15, 7 April 2003 (2003-04-07), pages 3071-3073, XP004415671 ISSN: 0040-4039
- CAMOUTSIS C: "Steroidal Oxazoles, Oxazolines, and Oxazolidines" JOURNAL OF HETEROCYCLIC CHEMISTRY, vol. 33, 1996, pages 539-557, XP002260229

## Description

### FIELD OF THE INVENTION

The present invention is directed to novel estrieno[3.2-b]/[3,4-c]pyrrole derivatives, pharmaceutical compositions containing them and their use in the treatment or prevention of disorders and diseases mediated by an estrogen receptor such as hot flashes, vaginal dryness, osteopenia, osteoporosis, hyperlipidemia, loss of cognitive function, degenerative brain diseases, cardiovascular diseases, cerebrovascular diseases, hormone sensitive cancers and hyperplasia (in tissues including breast, endometrium, and cervix in women and prostate in men), endometriosis, uterine fibroids, osteoarthritis; and as contraceptive agents either alone or in combination with a progestogen or progestogen antagonist. The compounds of the invention are selective estrogen receptor modulators.

### BACKGROUND OF THE INVENTION

Estrogens are a group of female hormones essential for the reproductive process and for the development of the uterus, breasts, and other physical changes associated with puberty. Estrogens have an effect on various tissues throughout a woman's body, not only those involved in the reproductive process, such as the uterus, breasts, and external genitalia, but also tissues in the central nervous system, bones, the liver, skin, and the urinary tract. The ovaries produce most of the estrogens in women's body.

Endogenous estrogens, such as 17b-estradiol and estrone, play a central role in the development of and maintenance of the female sex organs, mammary glands, and other sexual characteristics. In addition to their role as female sex hormone, estrogens are involved in the growth and function of a number of other tissues, such as the cardiovascular system, the central nervous system, and the skeleton, both in females and males. The significance of the estrogens in the development of the female reproductive system led to the development of a variety of compounds that interact with the estrogen receptors, such as contraceptives and agents for treatment of breast cancers. More recently, intensive efforts have focused on the selective estrogen receptor modulators for treatment and prevention of postmenopausal conditions, such as osteoporosis, coronary artery disease, depression and Alzheimer disease.

Menopause is defined as the permanent cessation of menses due to loss of ovarian follicular function and the almost termination of estrogen production. The midlife transition of menopause is characterized by a decrease in estrogen that provokes both short-term and long-term symptoms with the vasomotor, urogenital, cardiovascular, skeletal and central nervous systems, such as hot flushes, urogenital atrophy, increased risk of cardiovascular disease, osteoporosis, cognitive and psychological impairment, including an increased risk of cognitive disorders and Alzheimer's disease (AD).

Seventy-five percent of all women experience some occurrence of vasomotor symptoms associated with the onset of menopause such as body sweating and hot flushes. These complaints may begin several years before menopause and in some women may continue for more than 10 years either relatively constant, or as instant attacks without a definable, provoking cause.

Urogenital symptoms associated with the onset of menopause involving the vagina include a sensation of dryness, burning, itching, pain during intercourse, superficial bleeding and discharge, along with atrophy and stenosis. Symptoms involving the urinary tract include a burning sensation during urination, frequent urgency, recurrent urinary tract infections, and urinary incontinence. These symptoms have been reported to occur in up to 50% of all women near the time of menopause and are more frequent a few years after menopause. If left untreated, the problems can become permanent.

Heart attack and stroke are major causes of morbility and mortality among senior women. Female morbility from these diseases increases rapidly after menopause. Women who undergo premature menopause are at greater coronary risk than menstruating women of similar age. The presence of serum estrogen has a positive effect on serum lipids. The hormone promotes vasodilation of blood vessels, and enhances the formation of new blood vessels. Thus the decrease in serum estrogen levels in postmenopausal women results in adverse cardiovascular effect. Additionally, it is theorized that differences in the ability of blood to coagulate may account for the observed difference in the occurrence of heart disease before and after menopause.

The skeleton is under a continuous process of bone degeneration and regeneration in a carefully regulated interaction among the bone cells. These cells are directly affected by estrogen. Estrogen deficiency results in a loss of bone structure and a decrease of bone strength. Rapid loss of bone mass during the year immediately following menopause leads to postmenopausal osteoporosis and increased risk of fracture.

Estrogen deficiency is also one of the causes for the degenerative changes in the central nervous system and may lead to Alzheimer's disease and decline of cognition. Recent evidence suggests an association between estrogen, menopause and cognition. More particularly, it has been reported that estrogen replacement therapy and the use of estrogen in women may prevent the development of AD and improve cognitive function.

Hormone replacement therapy (HRT) - more specifically estrogen replacement therapy (ERT) - is commonly prescribed to address the medical problems associated with menopause, and also to help hinder osteoporosis and primary cardiovascular complications (such as coronary artery disease) in both a preventive and therapeutical manner. As such, HRT is considered a medical therapy for prolonging the average life span of postmenopausal women and providing a better quality of life.

ERT effectively relieves the climacteric symptoms and urogenital symptoms and has shown significant benefits in the prevention and treatment of heart disease in postmenopausal women. Clinical reports have shown that ERT lowered heart attack rates and mortality rates in populations that received ERT versus similar populations not on ERT. ERT initiated soon after menopause may also help maintain bone mass for several years. Controlled investigations have shown that treatment with ERT has a positive effect even in older women up to age of 75 years.

However, there are numerous undesirable effects associated with ERT that reduce patient compliance. Venous thromboembolism, gallbladder disease, resumption of menses, mastodynia and a possible increased risk of developing uterine and/or breast cancer are the risks associated with ERT. Up to 30% of women who were prescribed ERT did not fill the prescription, and the discontinuation rate is between 38% and 70%, with safety concerns and adverse effects (bloating and break-through bleeding) the most important reasons for discontinuation.

A new class of pharmacological agents known as Selective Estrogen Receptor Modulators or SERMs have been designed and developed as alternatives for HRT. Raloxifene, a nonsteroidal benzothiophere SERM is marketed in the US and Europe for the prevention and treatment of osteoporosis under the trademark of Evista®. Raloxifene has been shown to reduce bone loss and prevent fracture without adversely stimulating endometrial and mammary tissue, though raloxifene is somewhat less efficacious than ERT for protecting against bone loss. Raloxifene is unique and differs significantly from ERT in that it does not stimulate the endometrium and has the potential for preventing breast cancer. Raloxifene has also demonstrated beneficial estrogen agonist effects on cardiovascular risk factors, more specifically through a rapid and sustained decrease in total and low-density lipoprotein cholesterol levels in patients treated with raloxifene. In addition, raloxifene has been shown to reduce plasma concentration of homocysteine, an independent risk factor for arteriosclerosis and thromboembolic disease.

However, raloxifene has been reported to exacerbate symptoms associated with menopause such as hot flushes and vaginal dryness, and does not improve cognitive function in senior patients. Patients taking raloxifene have reported higher rates of hot flashes compared with either placebo or ERT users and more leg cramps than placebo users, although women who took ERT had a higher incidence of vaginal bleeding and breast discomfort than raloxifene or placebo users.

As yet, neither raloxifene nor any of the other currently available SERM compounds has been shown to have the ability to provide all the benefits of currently available ERT such as controlling postmenopausal syndrome and preventing AD, without causing adverse side effects such as increasing risk of endometrial and breast cancer and bleeding. Thus there exists a need for compounds which are selective estrogen receptor modulators and which provide all of the benefits of ERT while also addressing the vasomotor, urogenital and cognitive disorders or conditions associated with the decrease in systemic estrogen associated with menopause.

US 3 484 435 is exemplary of the an. This document discloses [2,3-d]-pyrazoics of steroid compounds of the estrane, 19-nor-pregnane and 19-nor-cholestane series. The compounds are claimed to exhibit estrogenic, cortical, anticholesterolemic and hypopbysis-blocking activity.

The Journal of Heterocyclic Chemistry (1996), 33, 539-557 is also exemplary of the art. This article discloses oxazole being condensed into the estrieno system.

### SUMMARY OF THE INVENTION

The present invention is directed to a compound of formula (I) or (II) 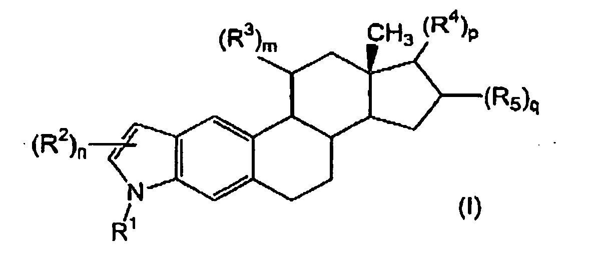 or 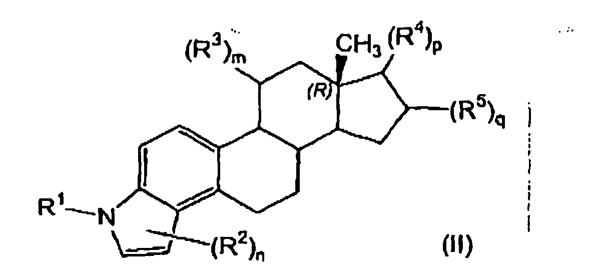 wherein
R¹ is selected from the group consisting of hydrogen, hydroxy. A. -O-A. C(O)-A and -SO₂-A;
n is an integer from 0 to 2;
each R² is independently selected from the group consisting of hydroxy- carboxy, halogen. -A, -O-A, -C(O)-A, -C(O)O-A. amino, (C1-C8)alkylamino- di(C1-C8)alkylamino, cyano, aminocarbonyl, (C1-C8)alkylaminocarbonyl, di(C1-C8)alkylaminocarbooyl, -SH, -S-A, -SO-A, -SO₂-A, -SO₂-NH₂, -SO₂-NH(C1-C8 alkyl) and -SO₂-N(C1-C8 alkyl)₂;
m is an integer from 0 to 2;
each R³ is independently selected from the group consisting of -A, -O-A, -S-A, -NH-A. -N(A)₂ and -C(O)-A;
p is an integer from 1 to 2;
each R⁴ is independently selected from the group consisting of hydroxy carboxy, cyano, -A, (C2-C8)alkenyl, -(C2-C8)alkynyl-A, (C2-C8)alkynyl, -(C2-C8)alkynyl-A, -O-A, -NH₂, NH(A), -N(A)₂, -N(A)-C(O)-A. -NH-C(O)-A, -C(O)-N(A)₂, -C(O)-NH₂, -C(O)-NH-A, -SO₂-N(A)₂, -SO₂-NH(A), -SO₂-NH₂- -N(A)-SO₂-A, -NH-SO₂-A, -C(O)O-A, -OC(O)H and -OC(O)-A;
alternatively, when p is 2. two R⁴ groups may be taken together as oxo or =N(OH):
q is an integer from 0 to 2;
each R⁵ is independently selected from the group consisting of hydroxy, carboxy, halogen, (C1-C8)alkyl, (C1-C8) alkoxy, (C3-C8)cycloalkyl and -C(O)-A; wherein the (C1-C8)alkyl group is optionally substituted with one or more substituents independently selected from halogen, hydroxy, carboxy or (C1-C8) alkoxy; wherein each A is independently selected from the group consisting of (C1-C8)alkyl, aryl, aralkyl, (C3-C8)cycloalkyl, heteroaryl and heterocycloalkyl; wherein the aryl, aralkyl, (C3-C8)eyeloalkyl, heteroaryl or heterocycloalkyl group is optionally substituted with one or more substituents independently selected from halogen, hydroxy, carboxy, (C1-C4)alkyl, (C1-C8) alkoxy, nitro, cyano, amino, (C1-C4)alkylamino or di ((C1-C4)alkyl) amino; wherein a heteroaryl is a five or six membered monocyclic aromatic ring structure containing at least one heteroatom selected from the group consisting of O, N and S or a nine of ten membered bicyclic aromatic ring structure containing at least one heteroatom selected from the group consisting of O, N and S, a heterocycloalkyl is a five to seven membered monocyclic, saturated or partially unsaturated ring structure containing at least one heteroatom selected from the group consisting of O, N and S or a nine to ten membered saturated, partially unsaturated or partially aromatic bicyclic ring system containing at least one heteroatom selected from the group consisting of O, N and S and an aryl is a carbocyclic aromatic group;
or a pharmaceutical acceptable salt thereof.

illustrative of the invention is pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

Exemplifying the invention is the use of the compounds or pharmaceutical compositions described above in the manufacture of a medicament for the treatment of a disorder mediated by one or more estrogen receptors.

Another example of the invention is the use of any of the compounds described herein in the preparation of a medicament for treating: (a) hot flashes, (b) vaginal dryness, (c) osteopenia, (d) osteoporosis, (e) hyperlipidemia, (f) loss of cognitive function, (g) a degenerative brain disorder, (h) a cardiovascular disease, (i) a cerebrovascular disease (j) breast cancer, (k) endometrial cancer, (I) cervical cancer, (m) prostate cancer, (n) benign prostatic hyperplasia, (o) endometriosis. (p) uterine fibroids, (q) osteoarthritis and for (r) contraception in a subject in need thereof.

### DETAILED DESCRIPTION OF THE INVENTION

The present invention is directed to compounds of formula (I) or (II) 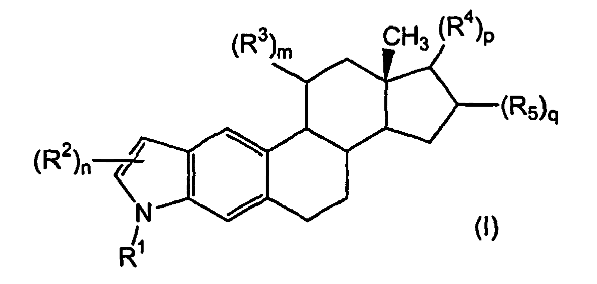 or 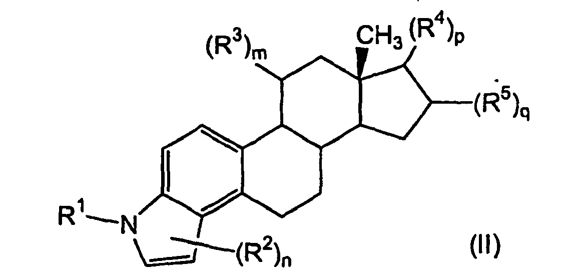 wherein R¹, n, R², m, R³, p, R⁴, q and R⁵ are as herein defined. The compounds of the present invention are modulators of an estrogen receptor, useful for the treatment and prevention of disorders associated with estrogen depletion, including, but not limited to hot flashes, vaginal dryness, osteopenia, osteoporosis, hyperlipidemia, loss of cognitive function, degenerative brain diseases, cardiovascular diseases and cerebrovascular diseases; for the treatment of hormone sensitive cancers and hyperplasia (in tissues including breast, endometrium, and cervix in women and prostate in men); for the treatment and prevention of endometriosis, uterine fibroids, and osteoarthritis; and as contraceptive agents either alone or in combination with a progestogen or progestogen antagonist.

In an embodiment of the present invention is a compound of formula (I). In another embodiment of the present invention is a compound of formula (II).

In another embodiment of the present invention R¹ is selected from the group consisting of hydrogen, hydroxy, (C1-C8) alkyl, aryl, aralkyl, -O-(C1-C8) alkyl, -O-aryl, -O-aralkyl, C(O)-A and -SO₂-A. In another embodiment of the present invention R¹ is selected from the group consisting of hydrogen and -SO₂-A. Preferably, R¹ is selected from the group consisting of hydrogen and -SO₂-(C1-C8) alkyl, more preferably R¹ is hydrogen or methylsulfonyl.

In an embodiment of the present invention A is selected from the group consisting of (C1-C8) alkyl, aryl and aralkyl; wherein the (C1-C8) alkyl, aryl or aralkyl group is optionally substituted with one or more, preferably one to three, more preferably on to two substituents independently selected from halogen, hydroxy, carboxy, (C1-C4) alkyl, (C1-C4) alkoxy, nitro, cyano, amino, (C1-C4) alkylamino, or di((C1-C4) alkyl)amino.

In an embodiment of the present invention n is an integer from 0 to 1, preferably n is 0. In an embodiment of the present invention R² is selected from the group consisting of carboxy, halogen, -A, -C(O)-A, -C(O)O-A, cyano, -S-A, -SO-A, -SO₂-A, -SO₂-NH₂. -SO₂-NH(alkyl) and -SO₂-N(alkyl)₂. Preferably R² is selected from the group consisting of -S-A, more preferably R² is selected from the group consisting of -S-((C1-C8) alkyl), more preferably still, R² is methylthio.

In an embodiment of the present invention m is an integer from 0 to 1, preferably m is 0.

In an embodiment of the present invention R³ is selected from the group consisting of -A, -O-A, -S-A, -NH-A and -C(O)-A.

In an embodiment of the present invention p is to 1. In an embodiment of the present invention R⁴ is selected from the group consisting of hydroxy and -O-C(O)-A. Preferably, R⁴ is selected from the group consisting of hydroxy, -O-C(O)-(C1-C8 alkyl), -O-C(O)-(C1-C8 alkyl)-CO₂H and alkynyl. More preferably, R⁴ is selected from the group consisting of hydroxy, n-butylcarbonyloxy, 1-carboxy-n-butylcabonyloxy and ethynyl.

In an embodiment of the present invention p is 2 and two R⁴ groups are taken together as oxo or =N(OH). Preferably, p is 2 and two R⁴ groups are taken together as oxo.

In an embodiment of the present invention R⁴ is selected from the group consisting of hydroxy, -NH₂, -NH(A), -NH(A), -C(O)NH₂, -C(O)-NH(A), -SO₂-NH₂, -SO₂-NH(A) and -OC(O)-A, and the R⁴ group is in a β-orientation. In another embodiment of the present invention R⁴ is selected from the group consisting of hydroxy, carboxy, cyano, -A, (C2-C8) alkenyl, -(C2-C8) alkenyl-A, (C2-C8) alkynyl, -(C2-C8) alkynyl-A, -O-A, -NH₂, -NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂NH(A), -SO₂-NH₂, -N(A)-SO₂-A, -NH-SO₂-A, -C(O)O-A, -OC(O)H and -OC(O)-A and the R⁴ group is in an a-orientation. In another embodiment of the present invention R⁴ is selected from the group consisting of hydrox, carboxy, cyano, C1-C4 alkyl, C1-C4 alkenyl and C1-C4 alkynyl and is in the α-orientation. In yet another embodiment of the present invention, two R⁴ groups are taken together as oxo or =N(OH).

In an embodiment of the present invention, q is an integer from 0 to 1, preferably q is 0.

In an embodiment of the present invention R⁵ is selected from the group consisting of carboxy, halogen, C1-C4 alkyl, and -C(O)-A. Preferably R⁵ is hydrogen.

Representative compounds of the formula (I) and (II) of the present invention are as listed in Table 1 and 2.

**Table 1**

| | | | |
|---|---|---|---|
| 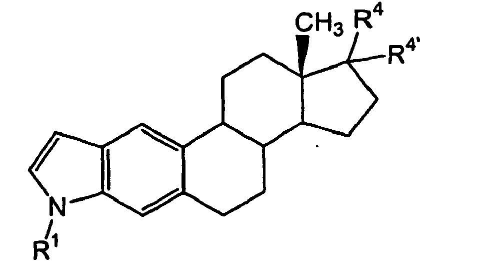 | | | |

| **ID No.** | **R¹** | **R⁴** | **R^{4'}** |
|---|---|---|---|
| 1 | methylsulfonyl | oxo | - |
| 3 | H | oxo | - |
| 7 | methylsulfonyl | β-hydroxy | H |
| 8 | H | β-hydroxy | H |
| 10 | H | β-hydroxy | a-ethynyl |
| 12 | H | β-[-OC(O)-n-butyl] | H |
| 13 | H | β-[OC(O)-n-butyl-COOH] | H |
| 14 | H | β-[-OC(O)-n-butyl-COO Na⁺] | H |

**Table 2**

| | | | | |
|---|---|---|---|---|
| 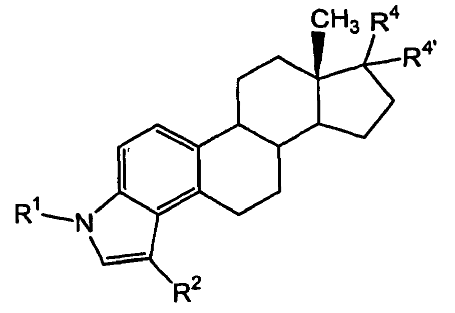 | | | | |

| **ID No.** | **R¹** | **R²** | **R⁴** | **R^{4'}** |
|---|---|---|---|---|
| 2 | methylsulfonyl | H | oxo | - |
| 4 | H | H | oxo | - |
| 5 | methylsulfonyl | H | oxo | - |
| 6 | H | H | β-hydroxy | H |
| 9 | H | H | β-hydroxy | α-ethynyl |
| 11 | H | H | β-[-OC(O)-n-butyl] | H |
| 15 | H | metylthio | oxo | - |

As used herein, unless otherwise noted, the term "halogen" shall mean chlorine, bromine, fluorine and iodine.

As used herein, unless otherwise noted, the term "alkyl" whether used alone or as part of a substituent group, include straight and branched carbon chains. For example, alkyl groups include methyl, ethyl, propyl, isopropyl, butyl, isobutyl, sec-butyl, t-butyl, pentyl and the like. Preferably, the alkyl group contains one to eight carbon atoms. Unless otherwise noted, "lower" when used with alkyl means a carbon chain composition of one to four carbon atoms.

As used herein, unless otherwise noted, the term "alkenyl" whether used alone or as part of a substituent group, shall include straight and branched chains containing at least one unsaturated double bond. For example, vinyl, propenyl or allyl, butenyl, buten-2-yl, buten-3-yl, 2-methyl-buten-2-yl, and the like. Preferably, the alkenyl group contains two to eight carbon atoms. Unless otherwise noted, "lower" when used with alkenyl means a carbon chain composition of two to four carbon atoms containing at least one unsaturated double bond.

As used herein, unless otherwise noted, the term "alkynyl" whether used alone or as part of a substituent group, shall include straight and branched chains containing at least one unsaturated triple bond. For example, ethynyl, propynyl, butyn-2-yl, and the like. Preferably, the alkynyl group contains two to eight carbon atoms. Unless otherwise noted, "lower" when used with alkynyl means a carbon chain composition of two to four carbon atoms containing at least one unsaturated triple bond.

As used herein, unless otherwise noted, "alkoxy" shall denote an oxygen ether radical of the above described straight or branched chain alkyl groups. For example, methoxy, ethoxy, n-propoxy, sec-butoxy, t-butoxy, n-hexyloxy and the like.

As used herein, unless otherwise noted, "aryl" shall refer to a carbocylic aromatic group such as phenyl, naphthyl, and the like.

As used herein, unless otherwise noted, "aralkyl" shall mean any lower alkyl group substituted with an aryl group such as phenyl, naphthyl and the like. For example, benzyl, phenylethyl, phenylpropyl, naphthylmethyl, and the like.

As used herein, unless otherwise noted, the term "cycloalkyl" shall mean any stable three to eight, preferably five to eight, more preferably five to six membered monocyclic, saturated ring system, for example cyclopropyl, cyclobutyl, cyclopentyl, cyclohexyl, cycloheptyl and cyclooctyl.

As used herein, unless otherwise noted, "heteroaryl" shall denote any five or six membered monocyclic aromatic ring structure containing at least one heteroatom selected from the group consisting of O, N and S, optionally containing one to three additional heteroatoms independently selected from the group consisting of O, N and S; or a nine or ten membered bicyclic aromatic ring structure containing at least one heteroatom selected from the group consisting of O, N and S, optionally containing one to four additional heteroatoms independently selected from the group consisting of O, N and S. The heteroaryl group may be attached at any heteroatom or carbon atom of the ring such that the result is a stable structure.
Examples of suitable heteroaryl groups include, but are not limited to, pyrrolyl, furyl, thienyl, oxazolyl, imidazolyl, purazolyl, isoxazolyl, isothiazolyl, triazolyl, thiadiazolyl, pyridyl, pyridazinyl, pyrimidinyl, pyrazinyl, pyranyl, furazanyl, indolizinyl, indolyl, isoindolinyl, indazolyl, benzofuryl, benzothienyl, benzimidazolyl, benzthiazolyl, purinyl, quinolizinyl, quinolinyl, isoquinolinyl, isothiazolyl, cinnolinyl, phthalazinyl, quinazolinyl, quinoxalinyl, naphthyridinyl, pteridinyl, and the like.

As used herein, the term "heterocycloalkyl" shall denote any five to seven membered monocyclic, saturated or partially unsaturated ring structure containing at least one heteroatom selected from the group consisting of O, N and S, optionally containing one to three additional heteroatoms independently selected from the group consisting of O, N and S; or a nine to ten membered saturated, partially unsaturated or partially aromatic bicyclic ring system containing at least one heteroatom selected from the group consisting of 0, N and S, optionally containing one to four additional heteroatoms independently selected from the group consisting of O, N and S. The heterocycloalkyl group may be attached at any heteroatom or carbon atom of the ring such that the result is a stable structure.
Examples of suitable heteroaryl groups include, but are not limited to, pyrrolinyl, pyrrolidinyl, dioxalanyl, imidazolinyl, imidazolidinyl, pyrazolinyl, pyrazolidinyl, piperidinyl, dioxanyl, morpholinyl, dithianyl, thiomorpholinyl, piperazinyl, trithianyl, indolinyl; chromenyl, 3,4-methylenedioxyphenyl, 2,3-dihydrobenzofuryl, and the like.

As used herein, the notation "*" shall denote the presence of a stereogenic center.

When a particular group is "substituted" (e.g., aryl, aralkyl, cycloalkyl, heterocycloalkyl, heteroaryl), that group may have one or more substituents, preferably from one to five substituents, more preferably from one to three substituents, more preferably still from one to two substituents, independently selected from the list of substituents.

With reference to substituents, the term "independently" means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a "phenylalkylaminocarbonylalkyl" substituent refers to a group of the formula

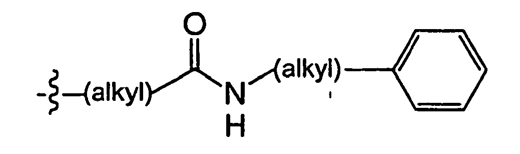

Unless other wise note, the position of substituents on the core structures of the compounds of formula (I) and (II) shall be denoted based on the following numbering system.

The position of substituent(s) on a compound of formula (I) shall be numbered such that the core of rings A, B, C and D are numbered based on accepted convention, and the fifth E ring positions are denoted with lower case letters a, b and c, beginning from the N atom, as follows:

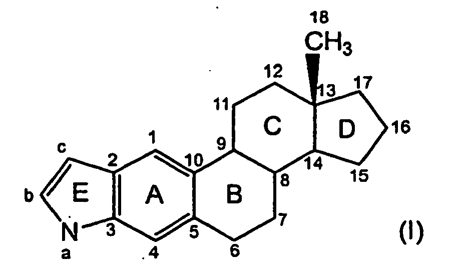

The position of substituent(s) on a compound of formula (II) shall be similarly denoted, as follows:

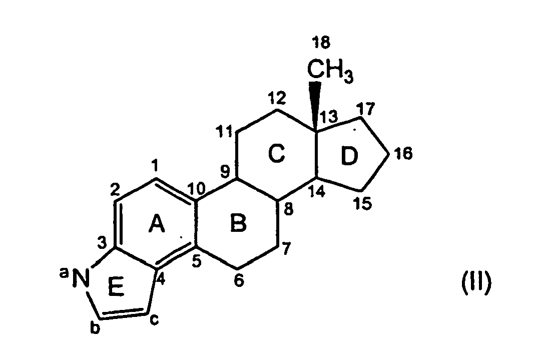

For the compounds of the present invention, each R⁴ substituent may be in an α- or a β- orientation, wherein in the α- orientation the R⁴ group is below the plane of the core molecule and in the β- orientation the R⁴ group is above the plane of the core molecule.

Abbreviations used in the specification, particularly the Schemes and Examples, are as follows:
- Ac: = Acetyl
- AcOH: = Acetic acid
- BINAP: = 2,2'-bis(diphenylphosphino)-1,1'-binaphthyl
- BnOH: = Benzyl alcohol
- Bu₃N: = Tributyl amine
- DCC: = N,N'-Dicyclohexylcarbodiimide
- DCM: = Dichloromethane
- DIC: = N,N'-Diisopropylcarbodiimide
- DIPEA: = Diisopropylethylamine
- DMAP: = 4-N,N-Dimethylaminopyridine
- DMF: = N,N-Dimethylformamide
- DMSO: = Dimethylsulfoxide
- DPPE: = 1,2-Bis(diphenylphosphino)-ethane
- DPPP: = 1,3-Bis(diphenylphosphino)-propane
- Et₂O: = Diethyl ether
- EtOAc: = Ethyl acetate
- EtOH: = Ethanol
- HEPES: = 4-(2-Hydroxyethyl)-1-piperazine ethane sulfonic acid
- LDA: = Lithium diisopropylamine
- LHMDS or LiHMDS: = Lithium bis(trimethylsilyl)amide
- mCPBA: = meta-chloroperoxybenzoic acid
- Me: = Methyl (i.e. -CH₃)
- MeOH: = Methanol
- NaO-t-Bu: = Sodium t-butoxide
- NBS: = N-bromosuccinimide
- NIS: = N-idoosuccinimide
- NMO: = N-methylmorpholine oxide
- PBS: = Phosphate buffered saline
- Pd(OAc)₂: = Palladium (II) acetate
- Pd₂(dba)₃: = Tris(dibenzylideneacetone) dipalladium (0)
- Pd(PPh₃)₂Cl₂: = Dichlorobis(triphenyphosphine) palladium (II)
- Ph: = Phenyl
- PhSO₂: = Phenyl sulfonyl
- PhS0₂Cl: = Phenyl sulfonyl chloride
- PPA: = Polyphosphoric acid
- PPh₃ or Ph₃P: = Tiphenyl phosphine
- PTSA: = p-Toluene sulfonic acid
- t-Bu: = t-Butyl
- t-BuoCI: = t-Butoxy chloride
- t-BuOH: = t-Butanol
- t-BuOK: = Potassium t-butoxide
- t-BuONa: = Sodium t-butoxide
- TEA or Et₃N: = Triethylamine
- TFA: = Trifluoroacetic acid
- THF: = Tetrahydrofuran

As used herein, the term "disease or disorder modulated or mediated by an estrogen receptor" shall mean any disease or disorder which is mediated by the estrogen α, any disease or disorder which is mediated by the estrogen β receptor or any disease or disorder which is mediated by both the estrogen α and estrogen β receptors. For example, hot flashes, vaginal dryness, osteopenia, osteoporosis, hyperlipidemia, loss of cognitive function, a degenerative brain disorder, cardiovascular disease, cerebrovascular disease breast cancer, endometrial cancer, cervical cancer, prostate cancer, benign prostatic hyperplasia (BPH), endometriosis, uterine fibroids, osteoarthritis and contraception.

As used herein, the term "degenerative brain disease" shall include cognitive disorder, dementia (regardless of underlying cause) and Alzheimer's disease.

As used herein, the term "cardiovascular disease" shall include elevated blood lipid levels, coronary arteriosclerosis and coronary heart disease.

As used herein, the term "cerebrovascular disease" shall include abnormal regional cerebral blood flow and ischemic brain damage.

As used herein, the term "progestogen antagonist" shall include mifepristone (RU-486), J-867 (Jenapharm / TAP Pharmaceuticals), J-956 (Jenapharm / TAP Pharmaceuticals), ORG-31710 (Organon), ORG-32638 (Organon), ORG-31806 (Organon), onapristone (ZK98299) and PRA248 (Wyeth).

The term "subject" as used herein, refers to an animal, preferably a mammal, most preferably a human, who is the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated. Wherein the present invention is directed to co-therapy comprising administration of one or more compound(s) of formula (I) or (II) and a progestogen or progestogen antagonist, "therapeutically effective amount" shall mean that amount of the combination of agents taken together so that the combined effect elicits the desired biological or medicinal response. For example, the therapeutically effective amount of co-therapy comprising administration of a compound of formula (I) and progestogen would be the amount of the compound of formula (I) and the amount of the progestogen that when taken together or sequentially have a combined effect that is therapeutically effective. Further, it will be recognized by one skilled in the art that in the case of co-therapy with a therapeutically effective amount, as in the example above, the amount of the compound of formula I and/or the amount of the progestogen or progestogen antagonist individually may or may not be therapeutically effective.

As used herein, the term "co-therapy" shall mean treatment of a subject in need thereof by administering one or more compounds of formula (I) or (II) with a progestogen or progestogen antagonist, wherein the compound(s) of formula (I) or (II) and the progestogen or progestogen antagonist are administered by any suitable means, simultaneously, sequentially, separately or in a single pharmaceutical formulation. Where the compound(s) of formula (I) or (II) and the progestogen or progestogen antagonist are administered in separate dosage forms, the number of dosages administered per day for each compound may be the same or different. The compound(s) of formula (I) or (II) and the progestogen or progestogen antagonist may be administered via the same or different routes of administration. Examples of suitable methods of administration include, but are not limited to, oral, intravenous (iv), intramuscular (im), subcutaneous (sc), transdermal, and rectal. Compounds may also be administered directly to the nervous system including, but not limited to, intracerebral, intraventricular, intracerebroventricular, intrathecal, intracisternal, intraspinal and / or peri-spinal routes of administration by delivery via intracranial or intravertebral needles and / or catheters with or without pump devices. The compound(s) of formula (I) or (II) and the progestogen or progestogen antagonist may be administered according to simultaneous or alternating regimens, at the same or different times during the course of the therapy, concurrently in divided or single forms.

As used herein, the term "composition" is intended to encompass a product comprising the specified ingredients in the specified amounts, as well as any product which results, directly or indirectly, from combinations of the specified ingredients in the specified amounts.

For use in medicine, the salts of the compounds of this invention refer to non-toxic "pharmaceutically acceptable salts." Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Compounds of formula (I) and (II) wherein R⁴ is oxo may be prepared according to the process outlined in Scheme 1.

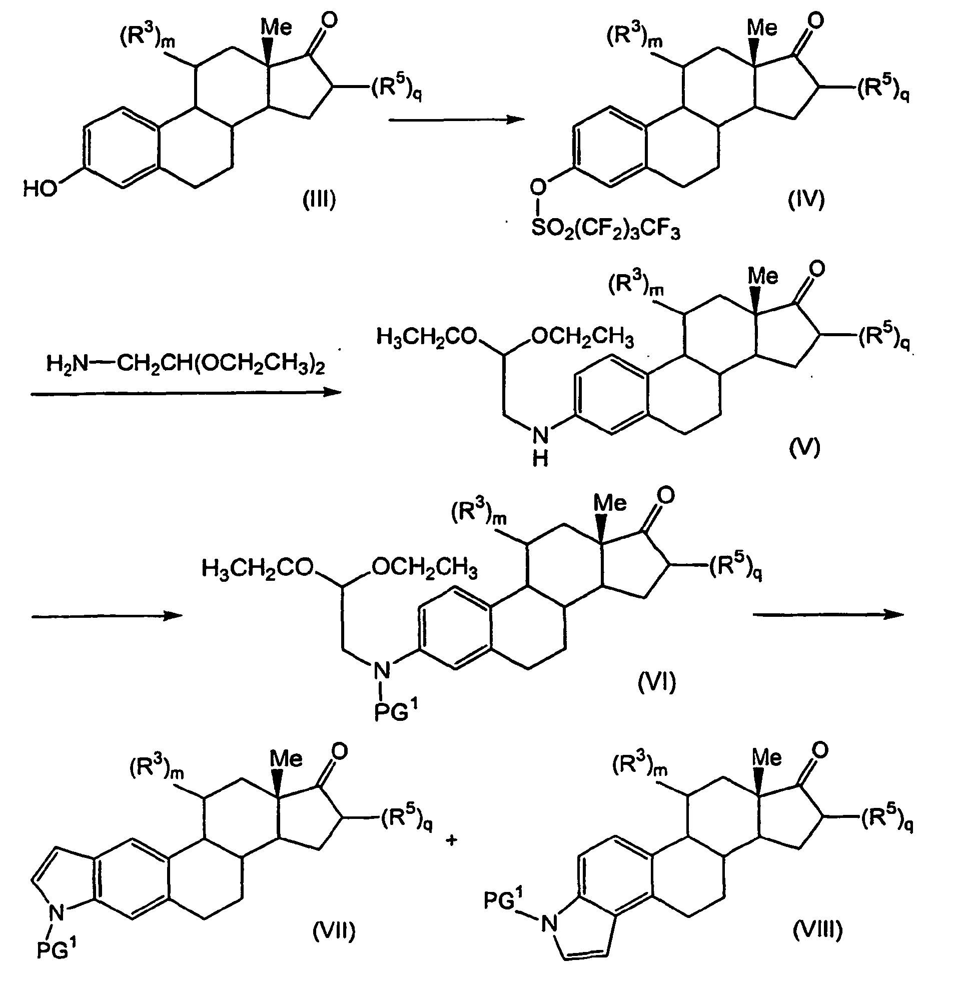

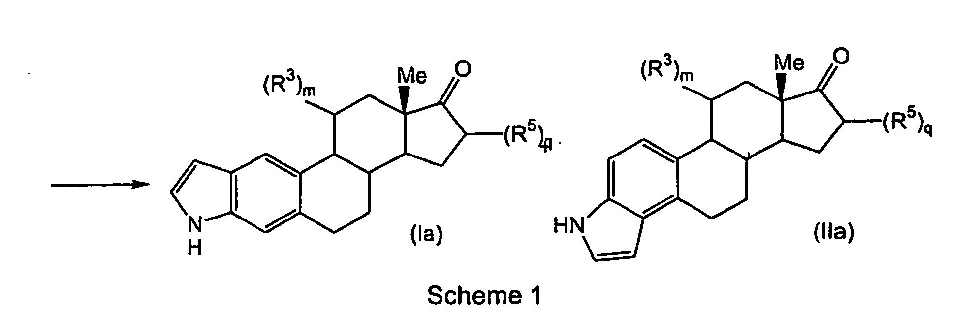

Accordingly, a suitably substituted compound of formula (III), a known compound or compound prepared by known methods, is reacted with 1,1,2,2,3,3,3-heptafluoro-propane-1-sulfonyl fluoride (i.e. CF₃(CF₂)₃SO₂F), a known compound, in the presence of a base such as TEA, DIPEA, pyridine, and the like, in an organic solvent such as DCM, THF, DMF, and the like, to yield the corresponding compound of formula (IV).

The compound of formula (IV) is reacted with 2,2-diethoxy-ethylamine, a known compound, in the presence of a phosphine ligand such as BINAP, DPPP, DPPE, and the like, in the presence of a catalyst such as Pd(OAc)₂, Pd₂(dba)₃, Pd(PPh₃)₂Cl₂, and the like, in the presence of a base such as sodium t-butoxide, Cs₂CO₃, K₂CO₃, and the like, to yield the corresponding compound of formula (V).

The compound of formula (V) is reacted with a suitable protecting agent, such as CH₃SO₂CI, PhSO₂CI, CH₃COCl, (CF₃CO)₂O, and the like, in the presence of a base such as TEA, DIPEA, pyridine, and the like, in an organic solvent such as DCM, THF, DMF, and the like, to yield the corresponding compound of formula (VI), wherein PG¹ is a protecting group such as CH₃SO₂, PhSO₂, Ac, CF₃CO, and the like, respectively.

The compound of formula (VI) is reacted with an acid such as PPA, PTSA, sulfuric acid, and the like, in an organic solvent such as toluene, xylene, and the like, to yield a mixture of the compound of formula (VII) and (VIII).

The compounds of formula (VII) and (VIII) are de-protected according to known methods, to yield the corresponding compounds of formula (Ia) and (IIa).

Preferably, the compounds of formula (la) and (IIa) are separated by known methods. Alternatively, the compounds of formula (VII) and (VIII) are separated by known methods, and then each is individually de-protected by known methods to yield the corresponding compounds of formula (Ia) and (IIa).

One skilled in the art will recognize that compounds of formula (I) and / or (II) wherein p is 2 and two R⁴ groups are taken together as =N(OH) may be prepared from the corresponding compound of formula (I) and / or (II) respectively, wherein p is 2 and two R⁴ groups are taken together as oxo by reacting the compound of formula (I) and / or (II) wherein p is 2 and two R⁴ groups are taken together as oxo with hydroxylamine, in an organic solvent such as ethanol, methanol, isopropanol, and the like.

Compounds of formula (I) and / or (II) wherein R⁴ is hydroxy may be prepared from the corresponding compound of formula (I) or (II) wherein R⁴ is oxo. As an example, Scheme 2 describes a process for the preparation of compounds of formula (I) wherein R⁴ is other than oxo.

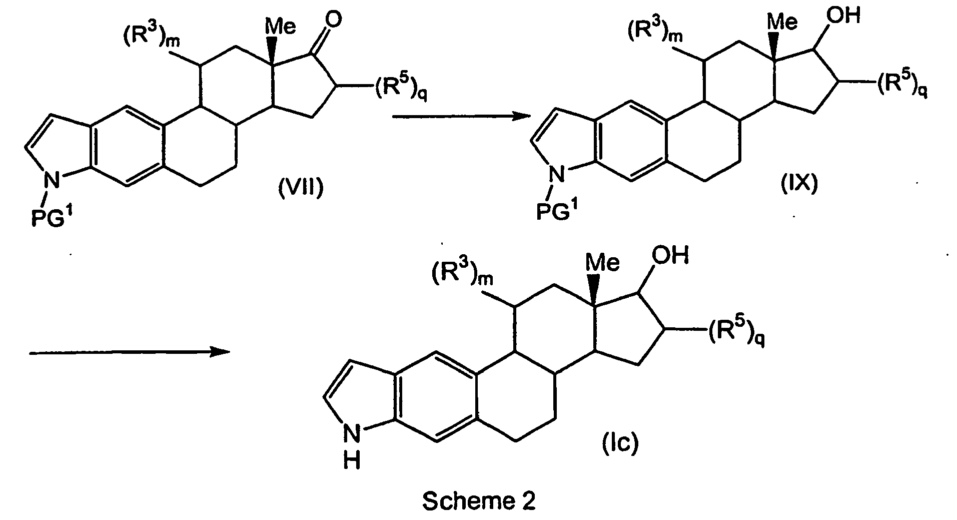

More specifically, a suitably substituted compound of formula (VII), prepared as in Scheme 1 above, is reduced with a reducing agent such as NaBH₄, LiBH₄, LiAIH₄, and the like, in a polar organic solvent such as methanol, ethanol, iso-propanol, and the like, to yield the corresponding compound of formula (IX).

The compound of formula (IX) is de-protected by known methods, to yield the corresponding compound of formula (Ic).

One skilled in the art will recognize that compounds of formula (II) wherein R⁴ is hydroxy may be similarly prepared according to the process outlined in Scheme 2 by substituting the compound of formula (VIII) for the compound of formula (VII).

Compounds of formula (I) wherein p is 2 and R⁴ is other than oxo or =N(OH) may be prepared according to the processes outlined in Scheme 3.

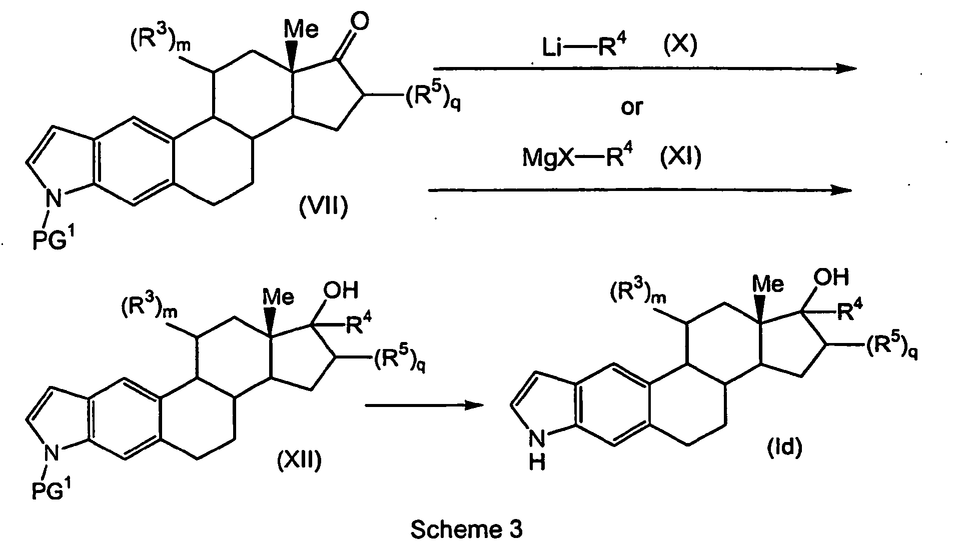

Accordingly, a compound of formula (VII), prepared as in Scheme 1 above, is reacted with a suitably substituted compound of formula (X), in the presence of an organo-lithium agent such as alkyl lithium, aryl lithium, alkenyl lithium, alkynyl lithium, and the like, in an organic solvent such as THF, diethyl ether, and the like, to yield the corresponding compound of formula (XII). The use of a compound of formula (X) is particularly preferred for introducing R⁴ groups selected from -A, alkenyl, alkynyl, -alkenyl-A and -alkynyl-A.

Alternatively, a compound of formula (VII), prepared as in Scheme 1 above, is reacted with suitably substituted compound of formula (XI), wherein X is Cl, Br or I, a Gringard agent such as alkyl magnesium halide, aryl magnesium halide, alkenyl magnesium halide, alkynyl magnesium halide, and the like, in an organic solvent such as THF, diethyl ether, dioxolane, and the like, to yield the corresponding compound of formula (XII). The use of a compound of formula (XI) is particularly preferred for introducing R⁴ groups selected from -A, alkenyl, alkynyl, -alkenyl-A and -alkynyl-A.

The compound of formula (XII) is de-protected according to known methods, to yield the corresponding compound of formula (Id).

One skilled in the art will recognize that that compounds of formula (11) may be similarly prepared according to the process outlined in Scheme 3 by substituting the compound of formula (VIII) for the compound of formula (VII).

Compounds of formula (I) wherein p is 1 and R⁴ is selected from -0-alkyl, -O-aralkyl, -O-cycloalkyl, -O-heterocycloalkyl or -O-C(O)-A may be prepared according to the process outlined in Scheme 4.

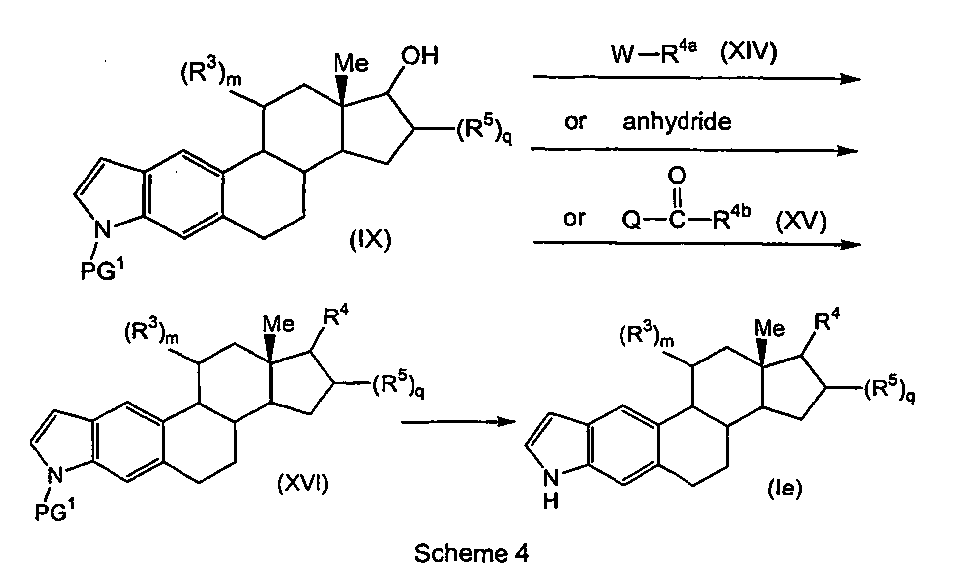

Accordingly, a compound of formula (IX), prepared as in Scheme 2 above, is reacted with suitably substituted compound of formula (XIV) wherein W is a leaving group such Cl, Br, I, tosylate, mesylate, triflate, and the like, and wherein R^{4a} is selected from the group consisting of alkyl, aralkyl, cycloalkyl and heterocycloalkyl, in the presence of a base such as pyridine, TEA, imidazole, and the like, in an organic solvent such as DCM, THF, DMF, and the like, optionally in the presence of a catalyst agent such as DMAP, and the like, to yield the corresponding compound of formula (XVI) wherein R⁴ is selected from -O-alkyl, -O-aralkyl, -O-cycloalkyl or -O-heterocycloalkyl.

Alternatively, a compound of formula (IX), prepared as in Scheme 2 above, is reacted with a suitably substituted anhydride (a compound of the formula R^{4a}-C(O)OC(O)-R^{4a}), wherein R^{4a} is selected from the group consisting of alkyl, aralkyl, cycloalkyl, heterocycloalkyl and -C(O)-A in the presence of a base such as pyridine, TEA, imidazole, and the like, in an organic solvent such as DCM, THF, diethyl ether, and the like, optionally in the presence of a catalyst DMAP, and the like, to yield the corresponding compound of formula (XVI) wherein R⁴ is selected from -O-C(O)-A.

Alternatively, a compound of formula (IX), prepared as in Scheme 2 above, is reacted with a suitably substituted compound of formula (XV), wherein Q is OH, Cl or Br, and wherein R^{4b} is selected from the group consisting of alkyl, aralkyl, cycloalkyl and heterocycloalkyl, to yield the corresponding compound of formula (XVI). Wherein the compound of formula (XV) Q is Cl or Br, the compound of formula (IX) is reacted with the compound of formula (XV) in the presence of a base such as pyridine, TEA, imidazole, and the like, in an organic solvent such as DCM, THF, diethyl ether, and the like, optionally in the presence of a catalyst DMAP, and the like, to yield the corresponding compound of formula (XVI). Wherein the compound of formula (XV) Q is OH, the compound of formula (IX) is reacted with the compound of formula (XV) in the presence of a coupling agent such as DCC, DIC, and the like, in an organic solvent such as THF, diethyl ether, DCM, acetonitrile, and the like, to yield the corresponding compound of formula (XVI) wherein R⁴ is selected from -O-C(O)-A.

The compound of formula (XVI) is de-protected according to known methods, to yield the corresponding compound of formula (le).

One skilled in the art will recognize that a compounds of formula (II) wherein p is 1 and R⁴ is selected from -O-alkyl, -O-aralkyl, -O-cycloalkyl, -O-heterocycloalkyl or -O-C(O)-A may be similarly prepared according to the process outlined in Scheme 4 with substitution of a compound of formula (XIII) 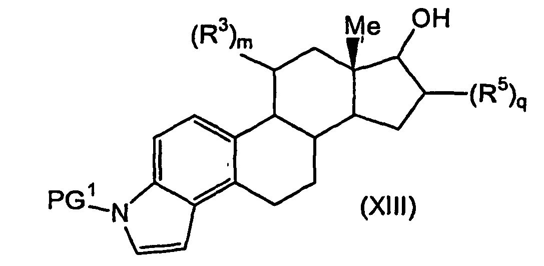 for the compound of formula (IX).

Compounds of formula (I) and (II) wherein n is 1 and the R² group is bound at the carbon atom adjacent to the N atom of the E ring may be prepared according to the process outlined in Scheme 5.

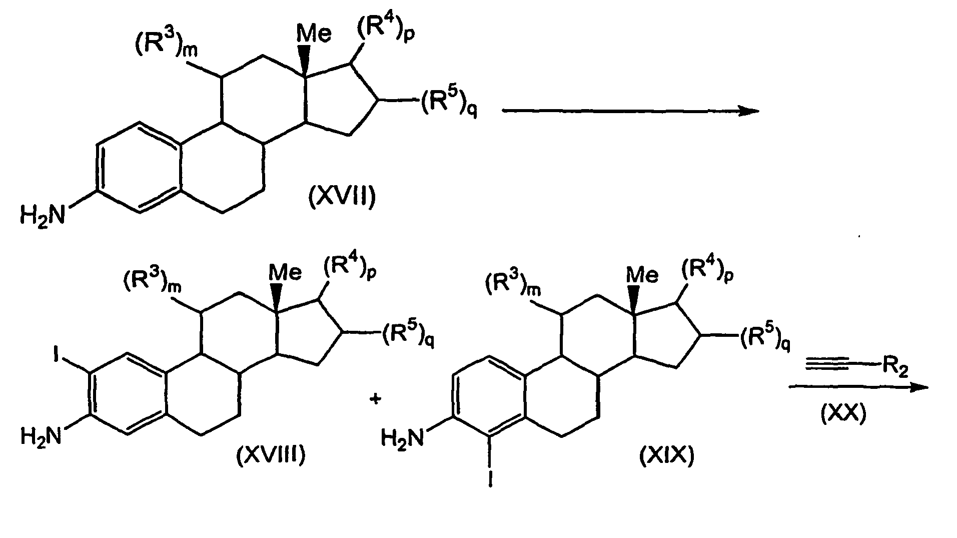

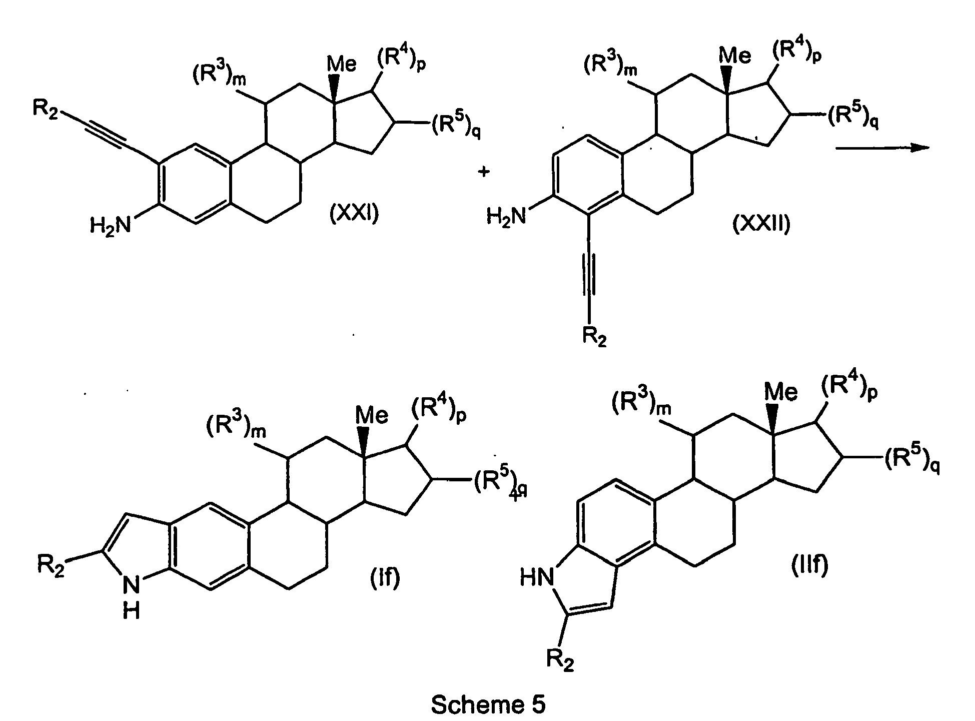

Accordingly, a suitably substituted compound of formula (XVII), a known compound or compound prepared by known methods, is reacted with an iodination agent such as NIS, iodine, ICI, and the like, in the presence of a coupling agent such as PTSA, and the like or a Lewis acid such as zinc chloride, BF₃ • etherate, and the like, in an organic solvent such as methanol, ethanol, THF, DMSO, DMF, and the like, to yield a mixture of the corresponding compounds of formula (XVIII) and (XIX).

Optionally, the compounds of formula (XVIII) and (XIX) are separated by known methods.

The mixture of compounds of formula (XVIII) and (XIX) is reacted with a suitably substituted compound of formula (XX), a known compound or compound prepared by known methods, in the presence of a coupling agent such as Pd(PPh₃)₂Cl₂, Pd(dba)₂, and the like, optionally in the presence of Cul, in an organic solvent such as TEA, DIPEA, pyridine, and the like, optionally in an organic solvent such as methylene chloride, DCM, THF, benzene, and the like, to yield a mixture of the corresponding compounds of formula (XXI) and (XXII).

Optionally, the compounds of formula (XXI) and (XXII) are separated by known methods.

The mixture of the compounds of formula (XXI) and (XXII) is reacted with Cul in an organic solvent such as benzene, toluene, DMF, and the like, at an elevated temperature in the range of about 50 to about 110°C, to yield the corresponding compounds of formula (If) and (IIf).

Preferably, the compounds of formula (If) and (IIf) are separated by known methods.

Compounds of formula (I) and (II) wherein n is 1 and the R² group is bound at the carbon atom adjacent to the N atom of the E ring may alternatively be prepared according to the process outlined in Scheme 6.

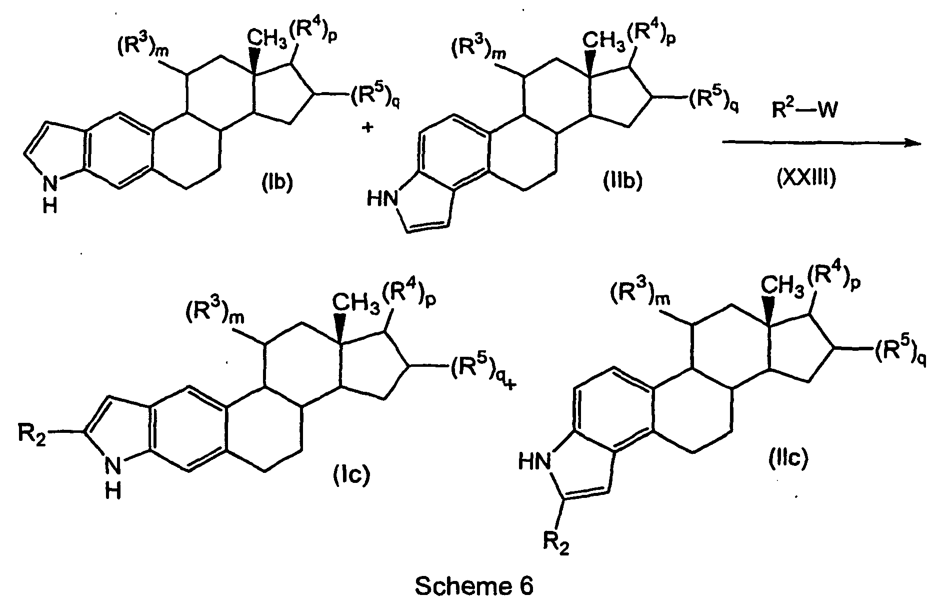

Accordingly, a suitably substituted compound of formula (Ib) or suitably substituted compound of formula (IIb) or mixture thereof (as exemplified herein), a known compound or compound prepared as described in the Schemes above, is reacted with a suitably substituted compound of formula (XXIII), wherein W is a leaving group such Cl, Br, I, tosylate, mesylate, triflate, and the like, a known compound or compound prepared by known methods, in the presence of a base such as LDA, LHMDS, t-butyl lithium, and the like, in an organic solvent such as THF, diethyl ether, dioxolane, and the like, to yield a mixture of the corresponding compounds of formula (Ic) and (IIc).

Preferably, the N atom on the E ring of the compound of formula (lb) and (IIb) is protected by known methods prior to reacting with the compound of formula (XXIII) and then de-protected by known methods.

Preferably, the compounds of formula (Ic) and (IIc) are separated by known methods.

Compounds of formula (I) and (II) wherein n is 1 and the R² group is one carbon atom away form the N (i.e. at the beta position) or wherein n is 2 may be prepared according to the process outlined in Scheme 7.

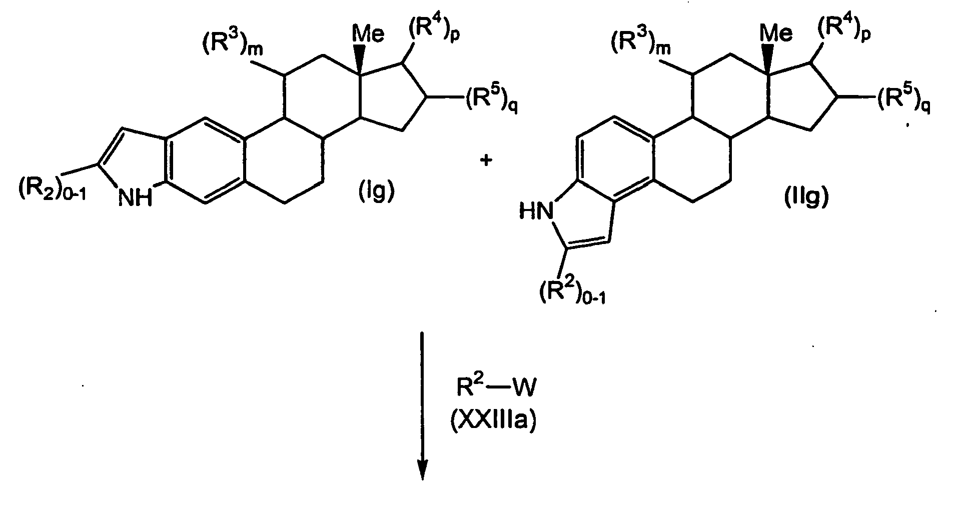

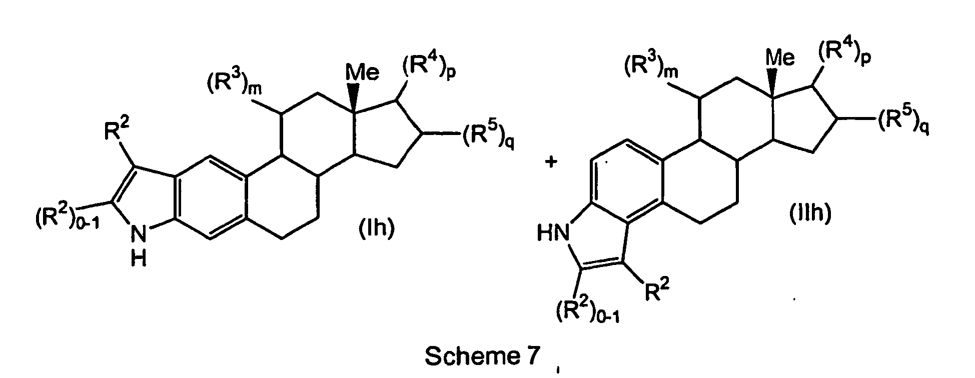

Accordingly, a suitably substituted compound of formula (Ig) or compound of formula (IIg) or mixture thereof (as exemplified in the Scheme herein) is reacted with a suitably substituted compound formula (XXIII), wherein W is a leaving group such Cl, BR, l, tosylate, mesylate, triflate, and the like, a known compound or compound prepared by known methods, in the presence of a catalyst such as AlCl₃, ZnCl₂, and the like, in an organic solvent such as methylene chloride, DCM, benzene, and the like, to yield a mixture of the corresponding compounds of formula (Ih) and (IIh).

Preferably, the compounds of formula (Ih) and (IIh) are separated by known methods.

Compounds of formula (II) wherein n is 1, R² is selected from -S-A, and the R² group is bound to the carbon atom adjacent to the N atom (i.e. at the alpha position), may be prepared according to the process outlined in Scheme 8.

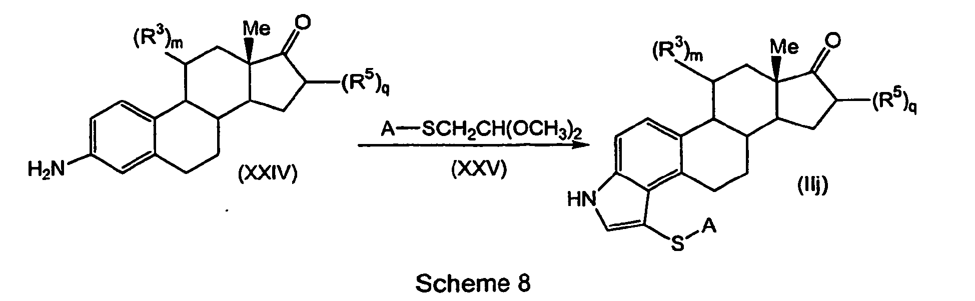

Accordingly, a compound of formula (XXIV), a known compound or compound prepared by known methods, is reacted with t-butoxy chloride and a compound of formula (XXV), a known compound or compound prepared by known methods, in the presence of a base such as TEA, DIPEA, pyridine, and the like, in an organic solvent such as DCM, chloroform, and the like, to yield the corresponding compound of formula (IIj).

Compounds of formula (I) wherein R¹ is selected from -OH or -O-A may be prepared according to the process outlined in Scheme 9.

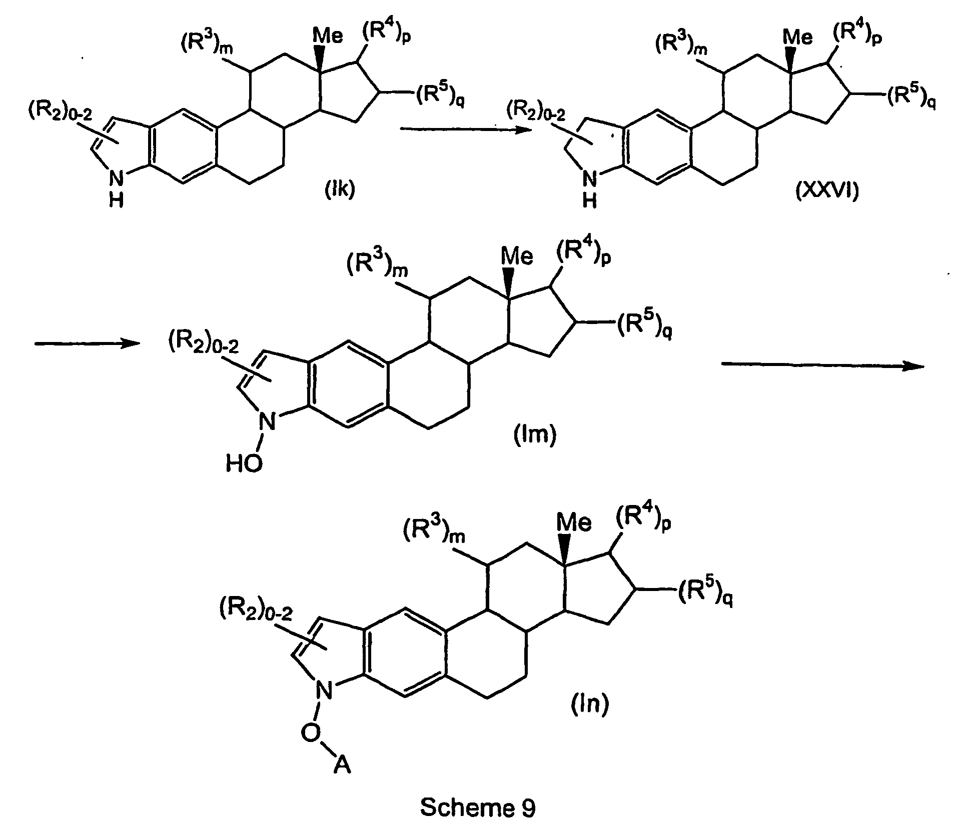

Accordingly, a suitably substituted compound of formula (Ik), is reacted with a reducing agent such as H₂, 1,4-cyclohexediene, triethylsilane, sodium cyanoboronhydride, borane, and the like, in the presence of an acid such as HCl, TFA, BF₃•etherate, and the like, in an organic solvent such as methanol, ethanol, THF and the like, to yield the corresponding compound of formula (XXVI).

The compound of formula (XXVI) is reacted with an oxidizing agent such as H₂O₂, NMO, and the like, in the presence of a catalysis such as NaH₂PO₂, Na₂WO₄, NalO₄, and the like, in an organic solvent such as methanol, pyridine, AcOH, and the like, to yield the corresponding compound of formula (Im), a compound of formula (I) wherein the R¹ group is -OH.

The compound of formula (Im) is optionally reacted (to displace the H atom on the OH group) with a suitably substituted reagent such as A-halogen, A-tosylate, A-mesylate, A-triflate, A-C(O)-halogen, A-SO₂-halogen, a symmetric anhydride (e.g. A-C(O)OC(O)-A), an asymmetric anhydride (e.g. A-C(O)OC(O)-A'), a cyclic anhydride, and the like, in the presence of a base such as NaH, t-BuOK, NaOH and the like, in an organic solvent such as THF, DCM, dioxlane, DMSO, DMF and the like, to the corresponding compound of formula (In), a compound of formula (I) wherein the R¹ group is O-A, -C(O)-A or -SO₂-A.

Compounds of formula (I) wherein R¹ is selected from -A, -C(O)-A or -SO₂-A may be prepared according to the process outlined in Scheme 10.

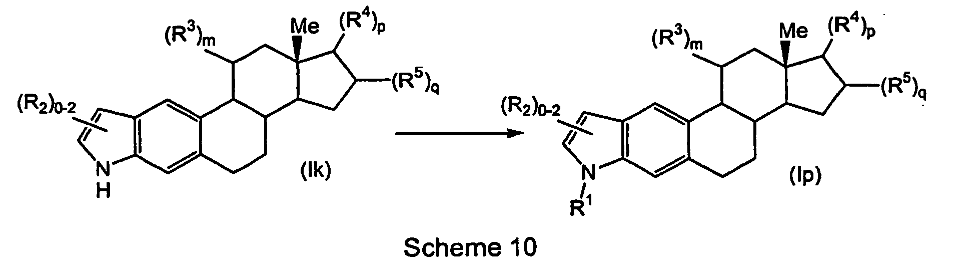

Accordingly, a suitably substituted compound of formula (Ik), is reacted with a suitably substituted reagent such as A-C(O)-halogen, A-SO₂-halogen, an asymmetric anhydride (e.g. A-C(O)OC(O)-A'), a symmetric anhydride (e.g. A-C(O)OC(O)-A), a cyclic anhydride, and the like, in the presence of a base such as NaH, t-BuOK, NaOH and the like, in an organic solvent such as THF, DCM, dioxlane, DMSO, DMF and the like, to yield the corresponding compound of formula (Ip), wherein the R¹ group is selected from -A (wherein A is other than aryl or heteroaryl), -C(O)-A or -SO₂-A.

For compounds of formula (1) wherein R¹ is -A and A is vinyl, aryl or heteroaryl, the compounds of formula (Ik) is reacted with a suitably substituted reagent such as vinyl-halide, (aryl or heteroaryl)-halide, (aryl or heteroaryl)-triflate, in the presence of a catalyst such as Pd(OAc)₂, Pd(PPh₃)₂Cl₂, Pd₂(dba)₃, Cul, CuBr and the like, in the presence of a base such as Cs₂CO₃, t-BuONa, CsF, K₂CO₃ and the like, in the presence of a ligand such as DPPP, DPPE, Ph₃P, BINAP and the like, in an organic solvent such as toluene, dioxlane, DMF and the like, to yield the corresponding compound of formula (Ip), wherein the R¹ is A and A is vinyl, aryl or heteroaryl.

One skilled in the art will recognize that compounds of formula (II) wherein R¹ is selected from -OH, -O-A, -A, -C(O)-A or -SO₂-A may be similarly prepared according to the processes outlined in Scheme 9 and 10 with substitution of a suitably substituted compound of formula (IIk) 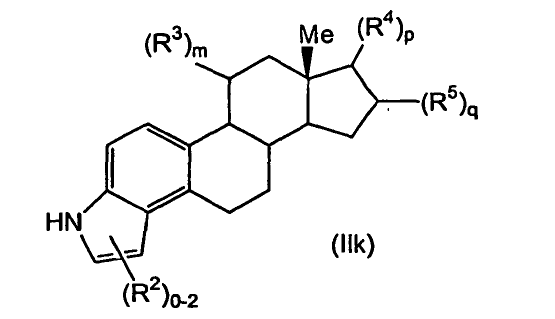 for the compound of formula (Ik). One skilled in the art will further recognize that a mixture of a compound of formula (Ik) and a compound of formula (IIk) may be substituted for the compound of formula (Ik) in Schemes 9 and 10 above, to yield a mixture of the corresponding compounds of formula (I) and (II) wherein R¹ is selected from -OH, -O-A, -A, -C(O)-A or SO₂-A. Wherein a mixture is reacted in Scheme 9 or 10, the resultant mixture of compounds of formula (I) and (II) is preferably separated, according to known methods.

Compounds of formula (I) wherein n is 1 and R² is selected from the group consisting of halogen, cyano, aryl, heteroaryl, -SO₂-NH₂, -SO₂-NH(alkyl) and -SO₂-N(alkyl)₂, and wherein the R² group is bound at the carbon atom adjacent to the N atom of the E ring, may be prepared according to the process outlined in Scheme 11.

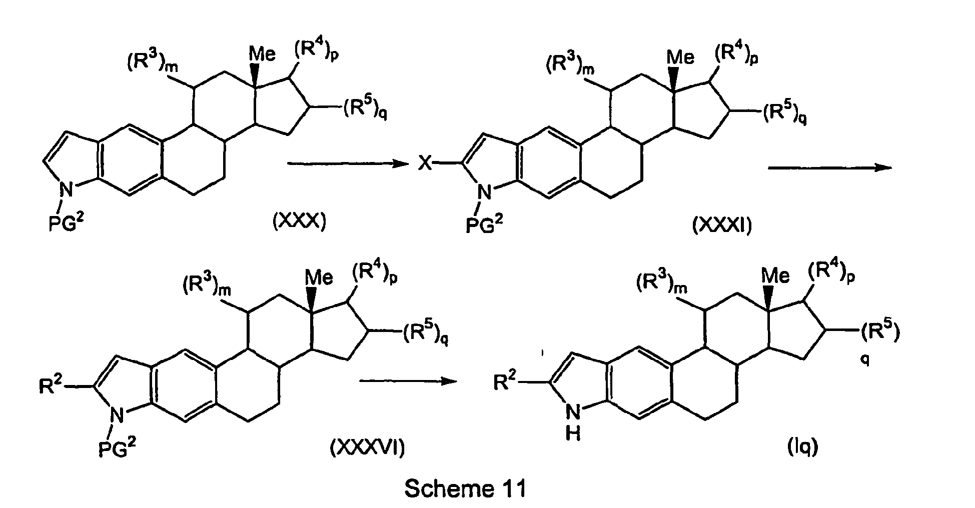

Accordingly, a suitably substituted compound of formula (XXX), wherein PG² is a suitably protecting group, is reacted with a halide source such as iodine, bromide, NIS, NBS, and the like, in the presence of a base such as LDA, LiHMDS, t-BuOK, and the like, in an organic solvent such as THF, diethyl ether, dioxlane, and the like, to yield the corresponding compound of formula (XXXI), wherein X is the corresponding halogen (for example when the halide source is iodine or NIS, then X is -I; when the halide source is bromine, NBS, then X is -Br).

The compound of formula (XXXI) is reacted with a coupling agent such as CuCN, and the like, optionally in the presence of a base such as diethylpropyl amine, N-methylpyrrolidone, and the like, in an organic solvent such as THF, DMF, DMSO, and the like, to yield the corresponding compound of formula (XXXVI), which is then de-protected by known methods; to yield the corresponding compound of formula (Iq), wherein the R² group is CN group.

Alternatively, the compound of formula (XXXI) is reacted with CO, in the presence of a catalyst such as Pd(OAc)₂, Pd(PPh₃)₂Cl₂, Pd₂(dba)₃, and the like, in the presence of a base such as Et₃N, Bu₃N, K₂CO₃ and the like, in the presence of a ligand such as DPPP, DPPE, Ph₃P, BINAP and the like, and then treated with a trapping agent such as H₂O, or an alcohol of the formula A-OH, such as MeOH, t-BuOH, phenol, and the like, in an organic solvent such as toluene, dioxlane, DMF and the like, to yield the corresponding compound of formula (XXXVI), which is then de-protected by known methods; to yield the corresponding compound of formula (Iq), wherein the R² group is carboxy (when the trapping agent is H₂O) or -C(O)-O-A (when the trapping agent is an alcohol).

Alternatively, the compound of formula (XXXI) is reacted with a suitably substituted organometallic agent such as an aryl or heteroaryl Grignard (a compound of the formula (aryl or heteroaryl)-Mg-halogen), a suitably substituted stannyl group (a compound of the formula (aryl or heteroaryl)Sn(alkyl)₃), a suitably substituted boronic acid (a compound of the formula (aryl or heteroaryl)-B(OH)₂) or a suitably substituted boronic ester (a compound of the formula (aryl or heteroaryl)-B(O-alkyl)₂), and the like, in the presence of a catalyst such as Pd(OAc)₂, Pd(PPh₃)₂Cl₂, Pd₂(dba)₃, and the like, a base such as Et₃N, Bu₃N, K₂CO₃ and the like, a ligand such as DPPP, DPPE, Ph₃P, BINAP and the like, in an organic solvent such as toluene, dioxlane, DMF and the like, to yield the corresponding compound of formula (XXXVI), which is then de-protected by known methods; to yield the corresponding compound of formula (Iq) wherein the R² group is aryl or heteroaryl.

Alternatively, the compound of formula (XXXI) is reacted with a suitably substituted nitrogen containing compound, in the presence of a catalyst such as Pd(OAc)₂, Pd(PPh₃)₂Cl₂, Pd₂(dba)₃, and the like, a base such as t-BuONa, K₃PO₄, Cs₂CO₃, K₂CO₃ and the like, a ligand such as DPPP, DPPE, Ph₃P, BINAP and the like, in an organic solvent such as toluene, dioxlane, DMF and the like, to yield the corresponding compound of formula (XXXVI), which is then de-protected by known methods; to yield the corresponding compound of formula (Iq) wherein the R² group is selected from amino, alkylamino, dialkylamino, aminocarbonyl, alkylaminocarbonyl or dialkylaminocarbonyl.

One skilled in the art will recognize that compounds of formula (I) wherein R² is -SO₂-NH₂, -SO₂-NH(alkyl) or -SO₂-N(alkyl)₂ may be similarly prepared by reacting a suitably substituted compound of formula (XXX) with chlorosulfonic acid, in an organic solvent such as THF, diethyl ether, DCM, DMF, and the like, and then displacing the chlorine atom by quenching with a suitably substituted amine.

Compounds of formula (I) wherein n is 1 and R² is selected from the group consisting of halogen, cyano, aryl, heteroaryl, -SO₂-NH₂, -SO₂-NH(alkyl) and -SO₂-N(alkyl)₂, and wherein the R² group is bound at the carbon atom one carbon atom removed from the N atom of the E ring, may be prepared similarly prepared according to the process outlined in Scheme 11. More particularly, the compound of formula (XXX) is reacted with a halide source such as iodine, bromide, NIS, NBS, and the like, in an organic solvent such as THF, diethyl ether, dioxlane, and the like, to yield the corresponding compound of formula (XXXII), 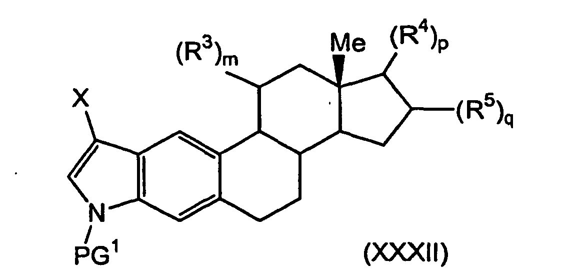 wherein X is the corresponding halogen (for example when the halide source is iodine or NIS, then X is -I; when the halide source is bromine, NBS, then X is -Br). The compound of formula (XXXII) is then reacted according to the process outlined above to displace the halogen atom with the desired R² group.

Compounds of formula (I) wherein n is 1 and R² is selected from the group consisting of -OH, -O-A, -S-A, -SO-A or SO₂-A may be prepared according to the process outlined in Scheme 12.

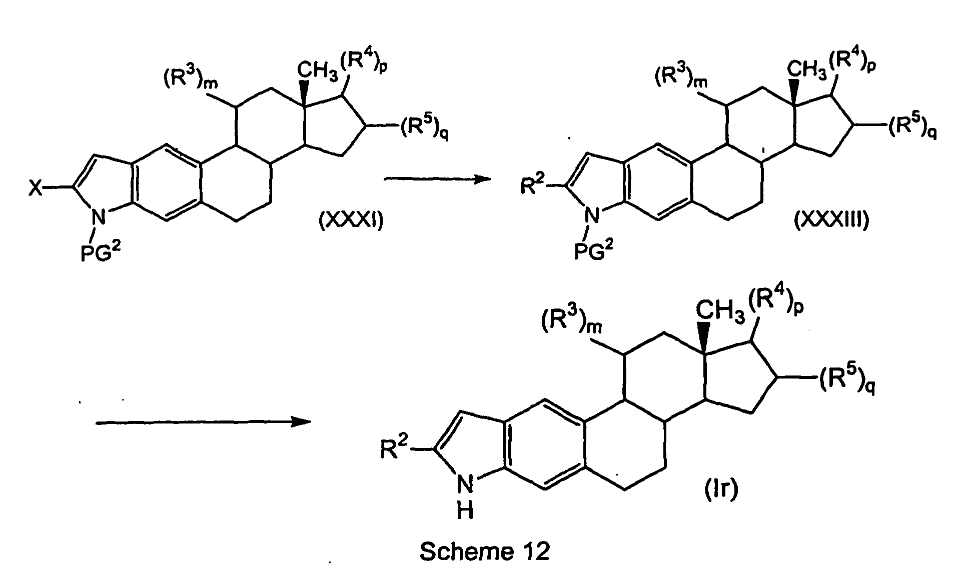

Accordingly, a suitably substituted compound of formula (XXXI), wherein PG² is a suitably protecting group, and wherein X is Cl, Br or I, is reacted with a coupling agent such as Cul, NiBr, and the like, in the presence of a suitably substituted alcohol, a compound of the formula A-OH, or a suitably substituted compound of the formula A-SH, in an organic solvent such as THF, DMF, toluene, dioxlane, and the like, optionally in the presence of a catalyst such as Pd(OAc)₂, Pd(PPh₃)₂Cl₂, Pd₂(dba)₃, and the like, to yield the corresponding compound of formula (XXXIII), wherein R² is -O-A or -S-A, respectively.

When in the compound of formula (XXXIII), R² is -S-A, the compound of formula (XXXIII) may be optionally reacted with an oxidizing agent such as oxone, mCPBA, H₂O₂, and the like, in a solvent such as MeOH, THF, H₂O, and the like, to yield the corresponding compound wherein the R² group is selected from -SO-A or -SO₂-A.

The compound of formula (XXXIII) is de-protected by known methods, to yield the corresponding compound of formula (Ir).

One skilled in the art will recognize that the processes outlined in Schemes 11 and 12 may be similarly applied to the preparation of compounds of formula (II) by substitution of a suitably substituted compound of formula (XXXIV) 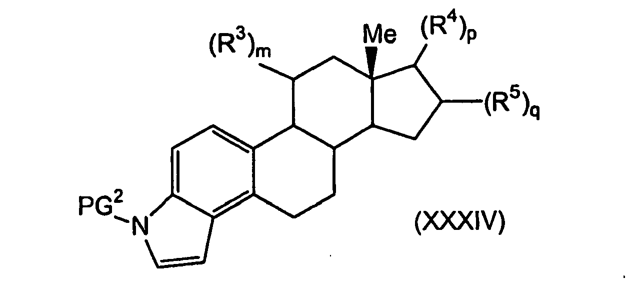 for the compound of formula (XXX) in Scheme 11 and substitution of a suitably substituted compound of formula (XXXV) 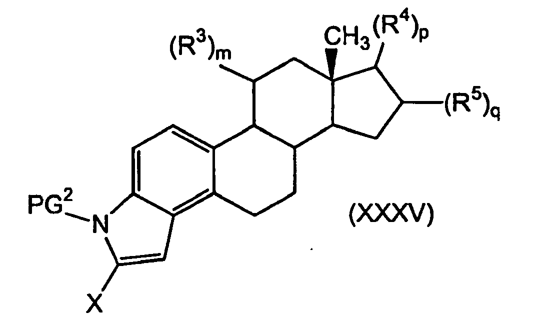 for the compound of formula (XXXI) in Scheme 12. One skilled in the art will further recognize that the processes in Schemes 11 and 12 may similarly be applied to yield a mixture of compounds of formula (I) and (II) by substitution of a mixture of suitably substituted compounds of formula (XXX) and (XXXIV) in Scheme 11 and a mixture of suitably substituted compounds of formula (XXXI) and (XXXV) in Scheme 12. Wherein the processes are applied to yield a mixture of compounds of formula (I) and (II), preferably, the compounds of formula (I) and (II) are separated by known methods.

One skilled in the art will further recognize that the processes described in the Schemes outlined herein may be carried out in any combination which will yield a compound of formula (I) or (II) with the desired substituent groups.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press,1973; and T.W. Greene & P.G.M. Wuts, Protective Groups in Organic Synthesis, John Wiley & Sons, 1991. The protecting groups may be removed at a convenient subsequent stage using methods known from the art.

The present invention includes within its scope prodrugs of the compounds of this invention. In general, such prodrugs will be functional derivatives of the compounds which are readily convertible *in vivo* into the required compound. Thus, in the methods of treatment of the present invention, the term "administering" shall encompass the treatment of the various disorders described with the compound specifically disclosed or with a compound which may not be specifically disclosed, but which converts to the specified compound *in vivo* after administration to the patient. Conventional procedures for the selection and preparation of suitable prodrug derivatives are described, for example, in "Design of Prodrugs", ed. H. Bundgaard, Elsevier, 1985.

The utility of the compounds of the instant invention to treat disorders mediated by an estrogen receptor may be determined according to the procedures described in Examples 18, 19, 20 and 21 herein.

The present invention therefore provides a method of treating disorders mediated by an estrogen receptor in a subject in need thereof which comprises administering any of the compounds as defined herein in a quantity effective to treat said disorder. The compound may be administered to a patient by any conventional route of administration, including, but not limited to, intravenous, oral, subcutaneous, intramuscular, intradermal and parenteral. The quantity of the compound which is effective for treating a disorder mediated by an estrogen receptor is between 0.01 mg per kg and 20 mg per kg of subject body weight.

The present invention also provides pharmaceutical compositions comprising one or more compounds of this invention in association with a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 5 to about 1000 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The method of treating a disorder mediated by an estrogen receptor described in the present invention may also be carried out using a pharmaceutical composition comprising any of the compounds as defined herein and a pharmaceutically acceptable carrier. The pharmaceutical composition may contain between about 5 mg and 1000 mg, preferably about 10 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms may include suitably flavored suspending or dispersing agents such as the synthetic and natural gums, for example, tragacanth, acacia, methyl-cellulose and the like. For parenteral administration, sterile suspensions and solutions are desired. lsotonic preparations which generally contain suitable preservatives are employed when intravenous administration is desired.

The compound of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phophatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyl-eneoxidepolylysine substituted with palmitoyl residue. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

The compounds of this invention may be administered in any of the foregoing compositions and according to dosage regimens established in the art whenever treatment of a disorder mediated by an estrogen receptor is required.

The daily dosage of the products may be varied over a wide range from 5 to 1,000 mg per adult human per day. For oral administration, the compositions are preferably provided in the form of tablets containing, 1.0, 5.0, 10.0, 15.0, 25.0, 50.0, 100, 250 and 500 milligrams of the active ingredient for the symptomatic adjustment of the dosage to the patient to be treated. An effective amount of the drug is ordinarily supplied at a dosage level of from about 0.01 mg/kg to about 20 mg/kg of body weight per day. Preferably, the range is from about 0.1 mg/kg to about 10 mg/kg of body weight per day, and especially from about 0.5 mg/kg to about 10 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation and the advancement of the disease condition. In addition, factors associated with the particular patient being treated, including patient age, weight, diet and time of administration, will result in the need to adjust dosages.

The following Examples are set forth to aid in the understanding of the invention, and are not intended and should not be construed to limit in any way the invention set forth in the claims which follow thereafter.

### Example 1

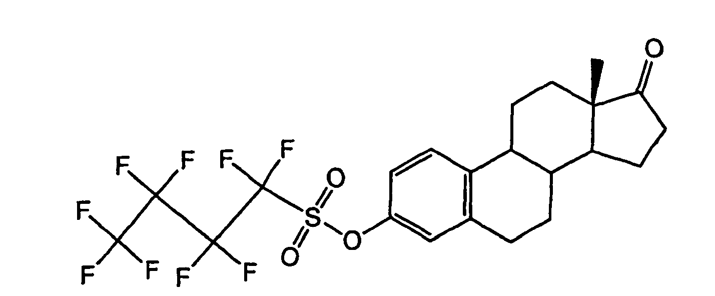

CF₃(CF₂)₃SO₂F (27.7 mmol, 4.97 mL) was added dropwise through a syringe into a solution of estrone (18.5 mmol, 5.0 g) and TEA (27.7 mmol, 3.90 mL) in CH₂Cl₂ (100 mL) at 0°C. The reaction mixture was slowly warmed to room temperature over 2 h. The solution was then washed with saturated NaHCO₃ and brine, then dried over anhydrous Na₂SO₄, filtered and concentrated to yield a white solid. The white solid was purified by flash chromatography using 4:1 hexanes/EtOAc to yield the title compound as a white solid.
¹H NMR (300 MHz, CDCl₃) δ 7.35 (d, J = 9.0 Hz, 1H), 7.05 (d, J = 9.0 Hz, 1H), 7.01 (s, 1H), 2.95 (m, J = 4.1 Hz, 2H), 2.42 (dd, J = 18.0, 8.8 Hz, 1H), 2.38 (m, 1H), 2.30 (m, 1H), 2.15 (dd, J = 17.6, 8.8 Hz, 1H), 2.10 ~ 1.99 (m, 3H), 1.77 ~ 1.51 (m, 6H), 0.98 (s, 3H)
MS (m/z) MH- (551).

### Example 2

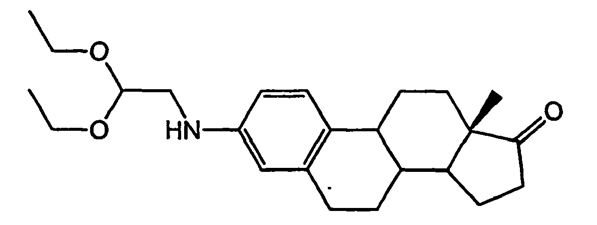

A three-neck flask was charged with Pd(OAc)₂ (0.0182 mmol, 4.1 mg), BINAP (0.02 mmol, 12 mg) and NaO-*t*-Bu (1.274 mmol, 122 mg). The reaction mixture was stirred in toluene (20 mL) for 10 min at 80°C. A mixture of NH₂CH₂CH(OCH₂CH₃)₂ (1.092 mmol, 159 µL) and the compound prepared as in Example 1 (0.91 mmol, 500 mg) in toluene (2 mL) was then slowly added dropwise via syringe to the reaction mixture. After addition, the mixture was heated at 80°C with stirring for 2 h. The reaction mixture was cooled to room temperature. Et₂O and water were added and the solution was partitioned between Et₂O and water. The Et₂O layer was washed with brine, dried over anhydrous Na₂SO₄ and concentrate to yield the title compound as a crude solid. The crude product was purified by column chromatography using 4:1 hexanes/EtOAc to yield the title compound as a colorless oil.
¹H NMR (300 MHz, CDCI₃) δ 7.12 (d, J = 8.8 Hz, 1 H), 6.53 (d, J = 8.8 Hz, 1 H) 6.38 (s, 1H), 4.68 (t, J = 6.0 Hz, 1 H), 3.80 (br, s, 1 H), 3.75 (m, 2H), 3.60 (m, 2H), 3.22 (d, J = 6.0 Hz, 2H), 2.91 (m, 2H), 2.50 (dd, J = 18.0, 8.8 Hz, 1H), 2.42 (m, 1H), 2.35 (m, 1 H), 2.20 (m, 1H), 2.15 ~ 1.94 (m, 3H), 1.72 ~ 1.38 (m, 6H), 1.25 (t, J = 7.6 Hz, 6H), 0.98 (s, 3H)
MS (m/z) M+Na (408), MH⁻ (384).

### Example 3

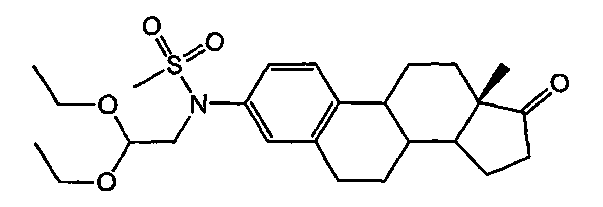

Pyridine (5.52 mmol, 446 µL) was added dropwise via syringe into a solution of the compound prepared as in Example 2 (3.68 mmol, 1.417 g) in CH₂Cl₂ (50 mL) at 0°C, followed by addition of CH₃SO₂Cl (4.42 mmol, 342 µL). The reaction mixture was then stirred and warmed to room temperature over 2 h. The solution was partitioned between CH₂CI₂ and saturated NaHC0₃. The organic layer was washed with brine and dried over anhydrous Na₂S0₄, filtered and concentrated to yield the title compound as a colorless oil. The product was sufficiently pure to be used in the next step without further purification.
¹H NMR (300 MHz, CDCI₃) δ 7.28 (d, J = 9.0 Hz, 1H), 7.15 (d, J = 9.0 Hz, 1 H), 7.10 (s, 1 H), 4.61 (t, J = 5.5 Hz, 1 H), 3.75 (d, J = 5.5 Hz, 2H), 3.62 (m, 2H), 3.54 (m, 2H), 3.05 (s, 3H), 2.91 (m, 1 H), 2.55 (m, 1 H), 2.40 (m, 1H), 2.22 (dd, J = 17.5, 8.0 Hz, 1H), 2.15 ~ 1.95 (m, 4H), 1.75 ~ 1.38 (m, 6H), 1.15 (t, J = 7.5 Hz, 6H), 0.94 (s, 3H)
MS (m/z) MH⁺ (464).

### Example 4 - (Compounds # 1 and #2)

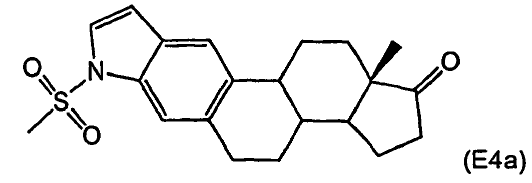

and

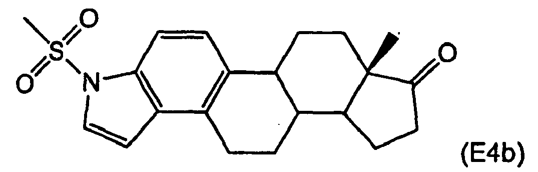

A mixture of the compound prepared in Example 2 (887 mg, 1.916 mmol) in toluene (15 mL) was treated with PPA (~1.0 g). The reaction mixture as then heated to reflux for 2 h. The reaction mixture was then cooled to room temperature, the solution was washed with water, saturated NaHC0₃ and rine and then dried over anhydrous Na₂S0₄. The resulting salt was filtered off and the solution was concentrated to afford a mixture of the title compounds as a clean oil. The oil was purified by chromatography (Biotage) using CH₂Cl₂ as eluent to yield the title compounds.
(E4a):
¹H NMR (300 MHz, CDCI₃) δ 7.68 (s, 1 H), 7.55 (s, 1 H), 7.36 (d, J = 3.2 Hz, 1 H), 6.67 (d, J = 3.2 Hz, 1 H), 3.20 - 3.00 (m, 2H), 3.10 (s, 3H), 2.62 ~ 2.50 (m, 2H), 2.45 (m, 1 H), 2.32 ~ 2.01 (m, 4H), 1.75 ~ 1.45 (m, 6H), 0.98 (s, 3H);
MS (m/z) MH⁺ (373).
(E4b):
¹H NMR (300 MHz, CDCl₃) δ 7.75 d, J = 7.5 Hz, 1H), 7.42 9d, J = 3.0 Hz, 1H), 7.39 (d, J = 7.5 Hz, 1H), 6.70 (d, J = 3.0 Hz, 1H), 3.21 ~ 3.02 (m, 2H), 3.11 (s, 3H), 2.60 - 2.38 (m, 3H), 2.28 - 1.98 (m, 4H), 1.83 - 1.48 (m, 6H), 0.98 (s, 3H)
MS (m/z) MH⁺ (373).

### Example 5 - (Compound #3)

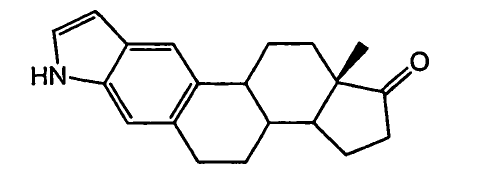

A mixture of Compound #1, prepared as in Example 3, (48 mg, 0.13 mmol) in 5% KOH in EtOH solution (1 mL) was heated at reflux for 6 h. The reaction mixture was then cooled to room temperature, the solvent removed *in vacuo* and the residue partitioned between EtOAc and water. The aqueous layer was extracted with EtOAc three times. The combined organic layer was then washed with saturated NH₄Cl, water and brine, then dried over anhydrous Na₂S0₄, filtered and concentrated to yield the title compound as a crude solid. The solid was purified by flash chromatography using CH₂Cl₂ to yield the title compound as white solid.
¹H NMR (300 MHz, CDCl₃) δ 8:02 (br, s1H), 7.58 (s, 1 H), 7.18 (s, 1H), 7.16 (d, J = 3.0 Hz, 1H), 6.45 (d, J = 4.5 Hz, 1H). 3.05 (m, J = 6.0 Hz, 2H), 2.58 ~ 2.35 (m, 3H), 2.25 - 1.97 (m, 4H), 1.78 ~ 1.45 (m, 6H), 0.92 (s, 3H)
MS (m/z) M+Na (316), MH⁺ (294).

### Example 6 - (Compound #4)

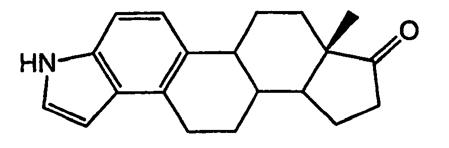

Compound #2, prepared as in Example 3 (54mg, 0.145 mmol) in 5% KOH in EtOH solution (1 mL) was heated at reflux for 6 h. The reaction mixture was then cooled to room temperature, the solvent was removed *in vacuo* and the residue was partitioned between EtOAc and water. The aqueous layer was extracted with EtOAc three times. The combined organic layer was then washed with sat. NH₄Cl, water and brine, then dried over anhydrous Na₂SO₄, filtered and concentrated to yield the title compound as a crude solid. The solid was purified by flash chromatography using CH₂Cl₂ to yield the title compound as a white solid.
¹H NMR (300 MHz, CDCl₃) δ 8.15 (br, s, 1H), 7.24 (s, 1H), 7.22 (s, 1H), 7.20 (d, J = 3.0 Hz, 1 H), 6.52 (d, J = 3.0 Hz, 1 H), 3.25 - 3.01 (m, 2H), 2.65 - 2.38 (m, 3H), 2.25 - 1.90 (m, 4H), 1.82 ~ 1.48 (m, 6H), 0.92 (s, 3H)
MS (m/z) MH⁺ (294).

### Example 7 - (Compound #7)

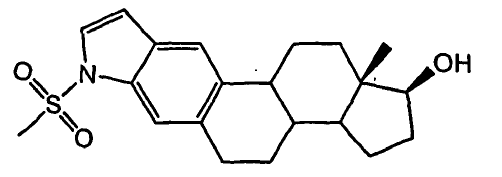

NaBH₄ (1.56 mmol, 59 mg) was added in one portion into a mixture of Compound #1, prepared as in Example 3 (58 mg, 0.156 mmol) in MeOH (2 mL) at 0°C. The reaction mixture was warmed to room temperature over 10 min. The solvent was removed *in vacuo* and the residue was partitioned between CH₂Cl₂ and water. The aqueous layer was extracted with CH₂Cl₂ twice. The, combined organic layer was then washed with brine, dried over anhydrous Na₂SO₄, filtered and concentrated to yield the title compound as a crude solid. The solid was purified by flash chromatography using 3:1 hexanes/EtOAc to yield the title compound as a white solid.
¹H NMR (300 MHz, CDCI₃) δ 7.65 (s, 1 H), 7.59 (s, 1 H), 7.32 (d, J = 4.0 Hz, 1H), 6.65 (d, J = 4.0 Hz 1H), 3.78 (t, J = 9.0 Hz, 1H), 3.10 (s, 3H), 3.12 ~ 3.01 (m, 2H), 2.82 - 2.78 (m, 1 H), 2.41 ~ 2.22 (m, 2H), 2.20 ~ 2.11 (m, 1 H), 1.98 ~ 1.92 (m, 1 H), 1.85 ~ 1.60 (m, 2H), 1.59 - 1.25 (m, 6H), 0.85 (s, 3H)
MS (m/z) MH⁺ (375).

### Example 8 - (Compound #5)

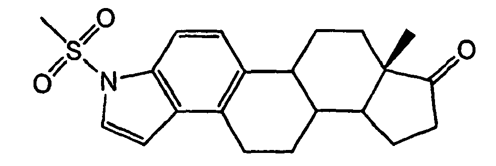

NaBH₄ (2.13 mmol, 81 mg) was added one portion into a mixture of Compound #2, prepared as in Example 3 (79 mg, 0.213 mmol) in MeOH (3 mL) at 0°C. The reaction mixture was warmed to room temperature over 10 min. The solvent was removed *in vacuo* and the residue was partitioned between CH₂Cl₂ and water. The aqueous layer was extracted with CH₂Cl₂ twice. The combined organic layer was then washed with brine, dried over anhydrous Na₂SO₄, filtered and concentrated to yield the title compound as a crude solid. The solid was purified by flash chromatography purification 3:1 hexanes/EtOAc to yield the title compound as a white solid.
¹H NMR (300 MHz, CDCl₃) δ 7.66 (d, J = 9.5 Hz, 1 H), 7.35 (d, J =3.5 Hz, 1 H), 7.28 (d, J = 9.5 Hz, 1 H), 6.65 (d, J = 3.5 Hz, 1 H), 3.70 (t, J = 9.0 Hz, 1H), 3.05 (s, 3H), 3.10 ~ 2.85 (m, 2H), 2.55 ~ 2.25 (m, 3H), 2.15 - 2.02 (m, 2H), 2.00 ~ 1.85 (m, 2H), 1.80 - 1.42 (m, 6H), 0.90 (s, 3H)
MS (m/z) MH⁺ (375).

### EXAMPLE 9 - (Compound #8)

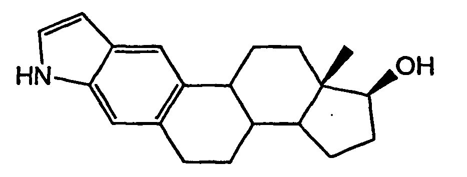

A mixture of Compound #7, prepared as in Example 7(51 mg, 0.137mmol) in 5% KOH in EtOH solution (2 mL) was refluxed for 2 h. The reaction mixture was then cooled to room temperature, the solvent was removed *in vacuo* and the residue was partitioned between CH₂Cl₂ and water. The aqueous layer was extracted with CH₂Cl₂ twice. The combined organic layer was then washed with saturated NH₄Cl, water and brine, then dried over anhydrous Na₂SO₄, filtered and concentrated to yield the title compound as a crude solid. The solid was purified by flash chromatography using 2:1 hexanes/EtOAc to yield the title compound as a white solid.
¹H NMR (300 MHz, CDCl₃) δ 8.01 (br, s, 1H), 7.68 (s, 1H), 7.14 (s, 1H), 7.12 (d, J = 3.5 Hz, 1H), 6.45 (d, J = 3.5 Hz, 1 H), 3.75 (t, J =8.5 Hz, 1H), 3.05 ~ 2.95 (m, 2H), 2.52 (m, 1H), 2.43 (m, 1H), 2.15 (m, 1 H), 2.05 ~ 1.88 (m, 2H), 1.80 ~ 1.58 (m, 2H), 1.55 ~ 1.28 (m, 6H), 0.85 (s, 3H)
MS (m/z) M+Na (318), MH⁺ (296).

### Example 10 - (Compound #6)

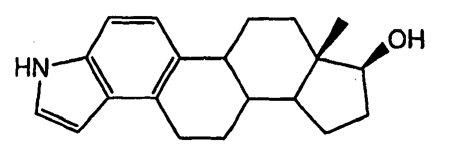

Compound #5, prepared as in Example 8 (71 mg, 0.190 mmol) in 5% KOH in EtOH solution (2 mL) was refluxed for 2 h. After the reaction was cooled down to room temperature, the solution was removed *in vacuo* and the residue was partitioned between CH₂Cl₂ and water. The aqueous layer was extracted with CH₂Cl₂ twice. The combined organic layer was then washed with saturated NH₄Cl, water and brine, then dried over anhydrous Na₂SO₄, filtered and concentrated to generate a clean solid. The solid was purified by flash chromatography using 2:1 hexanes/EtOAc to yield the title compound as white solid.
¹H NMR (300 MHz, CDCI₃) δ 8.05 (br, s, 1H), 7.15 (s, 1 H), 7.13 (s, 1H), 7.12 (d, J = 3.5 Hz, 1H), 6.45 (d, J = 3.5 Hz, 1H). 4.65 (t, J = 9.5 Hz, 1H), 3.10 ~ 2.90 (m, 2H), 2.40 ~ 2.28 (m, 2H), 2.20 ~ 2.08 (m, 1 H), 1.98 (m, 1H), 1.85 (m, 1H), 1.78 (m, 1 H), 1.65 (m, 1 h), 1.60 ~ 1.28 (m, 6H), 0.85 (s, 3H)
MS (m/z) M+Na (318), MH⁻ (294).

### Example 11 - (Compound #10)

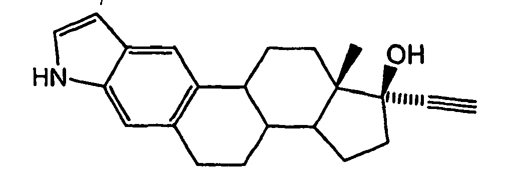

Lithium acetylide-ethylenedimine complex (46 mg, 0.523 mmol) was added into a mixture of Compound #1, prepared as in Example 4 (45 mg, 0.153 mmol) in dry dioxane (1 mL) and DMSO (1 mL) at room temperature. The reaction mixture was stirred for 1 h. The solvent was removed and then EtOAc and water were added. The mixture was partitioned between EtOAc and water. The organic layer was washed with water and brine, then dried over anhydrous Na₂SO₄, filtered and concentrated to yield the title compound as a crude yellow oil. The oil was purified by flash chromatography using 2:1 hexanes/EtOAc to yield the title compound as a yellowish oil.
¹H NMR (300 MHz, CDCl₃) δ 8.00 (br, s, 1 H), 7.60 (s, 1 H), 7.15 (s, 1H), 7.12 (d, J = 2.0 Hz, 1H), 6.48 (d, J = 2.0 Hz, 1 H), 2.98 - 2.92 (m, 2H), 2.95 (s, 1 H), 2.82 - 2.68 (m, 1 H), 2.50 - 2.25 (m, 2H), 2.21 ~ 2.05 (m, 1 H), 2.01 ~ 1.85 (m, 1 H), 1.78 ~ 1.52 (m, 2H), 1.50 ~ 1.25 (m, 6H), 0.80 (s, 3H)
MS (m/z) MH⁺ (320).

### Example 12 - (Compound #9)

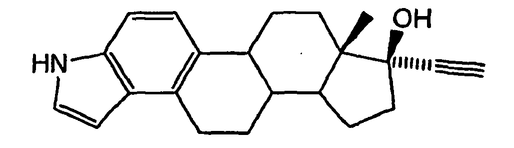

Lithium acetylide-ethylenedimine complex (69 mg, 0.784 mmol) was added into a mixture of Compound #2, prepared as in Example 4 (67 mg, 0.229 mmol) in dry dioxane (1 mL) and DMSO (1 mL) at room temperature. The reaction mixture was stirred for 1 h. The solvent was removed and then EtOAc and water were added. The mixture was partitioned between EtOAc and water. The organic layer was washed with water and brine, then dried over anhydrous Na₂SO₄, filtered and concentrated to yield the title compound as a crude yellow oil. The oil was purified by flash chromatography using 2:1 hexanes/EtOAc to yield the title compound as a yellowish oil.
¹H NMR (300 MHz, CDCl₃) δ 8.05 (br, s, 1 H), 7.26 (s, 1), 7.20 (s, 1H), 7.15 (d, J = 3.0 Hz, 1H), 6.48 (d, J = 3.0 Hz, 1 H), 3.10 - 2.95 (m, 2H), 2.60 (s, 1 H), 2.52 - 2.25 (m, 4H), 2.10 ~ 1.85 (m, 2H), 1.80 ~ 1.65 (m, 2H), 1.55 ~ 1.32 (m, 5H), 0.88 (s, 3H)
MS (m/z) MH⁺ (320).

### Example 13 - (Compound #12)

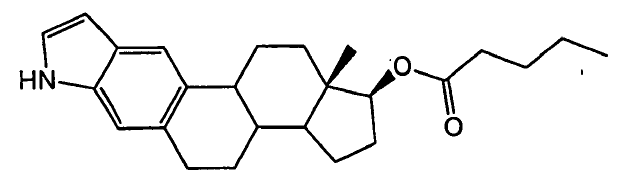

Pyridine (0.141 mmol, 12 µL) and catalytic amount of DMAP (1 mg) were added into in a mixture of 12a-methyl-1,2,3,3a,3b,4,5,7,10b,11,12,12a-dodecahydro-7-aza-dicylopenta[a,h]phenanthren-1-ol, a known compound, (38 mg, 0.129 mmol) in CH₂Cl₂ (2 mL) at 0°C, followed by addition of valeric choride (0.129 mmol, 16 µL). The reaction mixture was stirred at 0°C for 2 h and then warmed to room temperature. The mixture was partitioned between CH₂Cl₂ and water. The aqueous layer was extracted with CH₂Cl₂ twice. The combined organic layer was then washed with saturated NH₄Cl, water and brine, then dried over anhydrous Na₂SO₄, filtered and concentrated to yield the title compound as a crude solid. The solid was purified by flash chromatography using 3:1 hexanes/EtOAc to yield the title compound as a yellow solid.
¹H NMR (300 MHz, CDCl₃) δ 7.92 (br, s, 1H), 7.68 (s, 1 H), 7.15 (s, 1H), 7.14 (s, 1 H), 6.45 (s, 1 H), 4.72 (t, J = 8.5 Hz, 1 H), 3.05 (m, 2H), 2.48 - 2.40 (m, 2h), 2.35 (t, J = 7.5 Hz, 2H), 2.30 ~ 2.22 (m, 1 H), 2.00 ~ 1.85 (d, J = 9.5 Hz, 2H), 1.80 ~ 1.72 (m, 1 H), 1.70 ~ 1.52 (m, 1 H), 1.65 (t, J = 7.5 Hz, 2H), 1.50 ~ 1.25 (m, 6H), 1.36 (m, J = 7.5 Hz, 2H), 0.95 (t, J = 7.5 Hz, 3H), 0.90 (s, 3H)
MS (m/z) MH⁺ (381).

### Example 14 - (Compound #11)

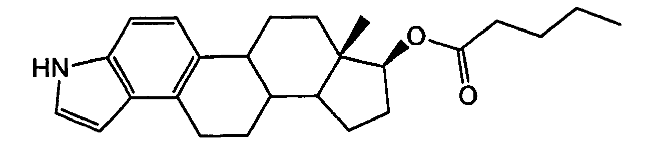

Pyridine (0.123 mmol, 10 µL) and catalytic amount of DMAP (1 mg) were added into in a mixture of 7a-methyl-3,5b,6,7,7a,8,9,10,10a,10b,11,12-dodecahydro-3-aza-dicyclopenta[a,l]phenanthren-8-ol, a known compound, (33 mg, 0.112 mmol) in CH₂CI₂ (2 mL)at 0°C, followed by addition of valeryl choride (0.123 mmol, 27 µL). The reaction mixture was stirred at 0°C for 2 h and then warmed to room temperature. The mixture was partitioned between CH₂CI₂ and water. The aqueous layer was extracted with CH₂CI₂ twice. The combined organic layer was then washed with saturated NH₄Cl, water and brine, then dried over anhydrous Na₂S0₄, filtered and concentrated to yield the title compound as a crude solid. The solid was purified by flash chromatography purification using 3:1 hexanes/EtOAc to yield the title compound as a yellow solid.
¹H NMR (300 MHz, CDCl₃) δ 8.12 (s, br, 1 H), 7.25 (d, J = 3.0 Hz, 1 H), 7.22 (d, J = 3.0 Hz, 1H), 7.22 (d, J = 3.0 Hz, 1 H), 7.20 (d, J = 3.5 Hz, 1 H), 6.52 (d, J = 3.0 Hz, 1H), 4.75 (t, J = 8.5 Hz, 1H). 3.10 ~ 2.98 (m, 2H), 2.48 ~ 2.40 (m, 2H), 2.33 (t, J = 8.0 Hz, 2H), 2.30 - 2.20 (m, 1 H), 2.11 ~ 2.02 (m, 1 H), 2.00 ~ 1.85 (m, 1 H), 1.80 ~ 1.66 (m, 1 H), 1.65 (t, J = 8.0 Hz, 2H), 1.60 - 1.28 (m, 5H), 1.40 (m, J = 8.0 Hz, 2H), 0.95 (t, J = 8.5 Hz, 3H), 0.85 (s, 3H)
MS (m/z). MH⁺ (381).

### Example 15 - (Compound #13)

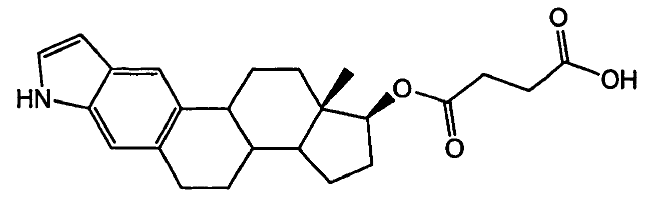

Pyridine (0.370 mmol, 30 µL) and catalytic amount of DMAP (10 mg) were added into in a mixture of 12a-methyl-1,2,3,3a,3b,4,5,7,10b,11,12,12a-dodecahydro-7-aza-dicylopenta[a,h]phenanthren-1-ol, a known compound, (68 mg, 0.231 mmol) in CH₂Cl₂ (2 mL) at 0°C, followed by addition of succinic anhydride (0.346 mmol, 35 mg). The reaction mixture was stirred at 0°C for 2 h and at room temperature overnight. The mixture was then partitioned between CH₂Cl₂ and water. The aqueous layer was extracted with CH₂Cl₂ twice. The combined organic layer was then washed with saturated NH₄Cl, water and brine, then dried over anhydrous Na₂SO₄, filtered and concentrated to yield the title compound as a crude solid. The solid was purified by flash chromatography using 2:1 hexanes/EtOAc to yield the title compound as a yellow solid.
¹H NMR (300 MHz, CDCl₃) δ 7.95 (br, s, 1H), 7.65 (s, 1H), 7.12 (s, 2H), 6.48 (s, 1 H), 4.72 (t, J = 7.0 Hz, 1 H), 3.10 (m, 2H), 2.80 ~ 2.58 (m, 4H), 2.50 ~ 2.32 (m, 2H), 2.30 ~ 2.15 (m, 1 H), 1.95 ~ 1.85 (d, J = 9.0 Hz, 2H), 1.80 (m, 1 H), 1.75 ~ 1.50 (m, 2H), 1.48 ~ 1.30 (m, 5H), 0.85 (s, 3H)
MS (m/z) MH⁺ (396), MH⁻ (394).

### Example 16 - (Compound #14)

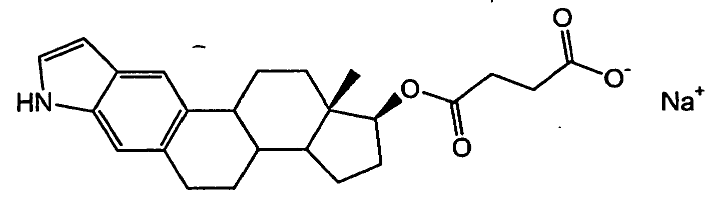

A solution of NaOH (0.0572 mmol, 1.0 N in THF solution) was added to Compound #13, prepared as in Example 15 (26 mg, 0.066 mmol). The mixture was stirred at room temperature for 1 h. The solvent was removed *in vacuo* to yield the title compound as a white solid. The solid was dried at 50°C under vacuum for 10 h to yield the title compound as a white solid.
¹H NMR (300 MHz, CDCl₃) δ 7.41 (s, 1H), 7.12 (d, J = 3.0 Hz, 1 H), 7.08 (s, 1H), 6.35 (d, J = 3.0 Hz, 1 H), 4.68 (t, J = 8.5 Hz, 1 H), 2.98 (m, 2H), 2.70 - 2.48 (m, 4H), 2.40 - 2.32 (m, 1 H), 2.32 ~ 2.18 (m, 1 H), 2.20 ~ 2.12 (m, 1 H), 1.98 ~ 1.85 (m, 2H), 1.80 - 1.62 (m, 1H), 1.60 ~ 1.48 (m, 1 H), 1.48 ~ 1.25 (m, 6H), 0.90 (s, 3H)

### Example 17 - (Compound #15)

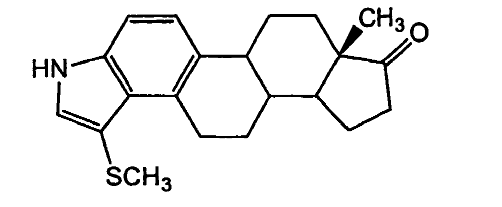

To a vigorously stirred solution of 3-Amino-13-methyl-6,7,8,9,11,12,13,14,15,16-decahydro-cyclopenta[a]phenanthren-17-one (101 mg, 0.37 mmoL) dichloromethane (5mL) at -65°C was added dropwise a soution of *t*-BuOCl (0.37 mmoL, 40 mg) in DCM (1 mL). After 5 min, 1,1-dimethoxy-2-methylsulfanyl-ethane (0.37 mmoL, 50 µL) dissolved in DCM (0.5 mL) was added to the reaction, followed by addition of Et₃N (0.37 mmoL, 52 µL). The reaction mixture was then stirred for 1 h. To the reacted mixture was added DCM (10 mL), the resulting mixture was washed with 1 N HCI, saturated NaHCO₃ and brine, then dried over anhydrous Na₂SO₄, filtered and concentrated to yield a crude product. The crude material was purified using column chromatography and a 3:1 hexanes:EtOAc mixture as eluent to yield the title compound as a pale solid.
¹H NMR (300 MHz, CDCl₃) δ 8.10 (br, s, 1 H), 7.20 (d, J = 8.5 Hz, 1 H), 7.15 (d, J = 8.5 Hz, 1H), 6.52 (d, J = 2.5 Hz, 1 H), 3.25 ~ 3.01 (m, 2H), 2.65 - 2.38 (m, 3H), 2.50 (s, 3H), 2.25 - 1.90 (m, 4H), 1.82 ~ 1.48 (m, 6H), 0.92 (s, 3H)
MS (m/z) MH⁺ (340).

### Example 18

### Estrogen Receptor α Flash Plate Assay

This assay monitors binding of radiolabeled estrogen to the estrogen receptor. It was performed on a BioMek 2000 (Beckman). Plates were read in a scintillation counter (Packard TopCount), with decreased counts an indication of binding of a compound to the receptor. The assay was run according to the procedure described by Allan, et al., Anal. Biochem. (1999), 275(2), 243-247.

On day one, 100 µL of Estrogen Screening Buffer (ESB, Panvera) containing 5mM dithiothreitol (DTT, Panvera), 0.5 µg mouse anti-estrogen receptor monoclonal antibody (SRA-1010, Stressgen) and 50 ng purified human estrogen receptor α (nER-α, Panvera) were added to each well of a 96 well FlashPlate Plus plate crosslinked with goat anti-mouse antibodies (NEN Life Sciences). The plate was sealed and incubated at 4°C overnight.

On day two, each well was washed three times with 200 µL PBS, pH 7.2, at room temperature. To each well was then added 98 µL radiolabeled estrogen (0.5 nM, which equals 6 nCi for a 120 Ci/mmol batch, Amersham), diluted in ESB and 5mM dithiothreitol (DTT). To individual wells were then added 2.5 µL test compound diluted in 30% (v/v) dimethyl sulfoxide/50 mM HEPES, pH 7.5. The wells were mixed three times by aspiration, the plate sealed and incubated at room temperature for one hour. The wells were then counted for 1 min in a TopCount scintillation counter (Packard).

### Example 19

### Estrogen Receptor β Fluorescence Polarization Assay

This assay monitors binding of a fluorescent analog of estrogen (Fluormone ES2, Panvera) to the estrogen receptor. Plates were read in a fluorometer that can be set to polarization mode. A decrease in fluorescence relative to vehicle control is an indication of binding of a compound to the receptor.

It is crucial to avoid introduction of air bubbles into the reaction in each well of the 96 well plate throughout this procedure. (Bubbles on the surface of the reaction disrupt light flow, affecting the polarization reading.) However, it is also crucial to effectively mix the reaction components upon addition to the well.

On ice, a 2X standard mixture of Assay Buffer (Panvera), 10 nM DTT and 40 nM ES2 was prepared. On ice, a 2X reaction mixture of Assay Buffer (Panvera), and 20 nM human estrogen receptor β (hER-β, Panvera) and 40 nM ES2 was also prepared.

Dilutions of test compound were prepared in 30% (v/v) dimethyl sulfoxide/50 mM HEPES, pH 7.5. At this point, the dilutions were 40X the final required concentration.

The standard mixture at 50 µL was then added to each well. The reaction mixture at 48 µL was added to all wells. The compound dilution at 2.5 µL was added to the appropriate wells. The reaction mixtures were mixed using a manual pipette, a roll of aluminum foil adhesive cover was placed on the plate and the plate incubated at room temperature for 1 hour.

Each well on the plate was then read in an LjL Analyst with an excitation wavelength of 265 nm and an emission wavelength of 538.

Representative compound of the present invention were tested according to the procedure described above for binding to the Estrogen Receptor α and Estrogen Receptor β as described in Examples 18 and 19 above, with results as listed in Table 3.

**Table 3**

| **ID No.** | **ERα Binding IC₅₀ (µM)** | **ERα at 1µM % Inhibition** | **ERβ Binding IC₅₀ (µM)** | **ERβ at 1 µM % Inhibition** |
|---|---|---|---|---|
| 1 | | 11 | | 6.2 |
| 2 | | 45 | | 27 |
| 3 | 2.2 | | >10 | |
| 4 | 1.4 | | 0.61 | |
| 6 | 0.042 | | 0.091 | |
| 7 | | 19 | | 14 |
| 8 | 0.040 | | 0.15 | |
| 12 | | 95 | | 96 |
| 13 | 0.92 | | 0.61 | |
| 15 | | 81 | | 66 |

### Example 20

### MCF-7 Cell Proliferation Assay

This assay was run according to the procedure described by Welshons, et al., (Breast Cancer Res. Treat., 1987, 10(2), 169-75), with minor modification.

Briefly, MCF-7 cells (from Dr. C. Jordan, Northwestern University) were maintained in RPMI 1640 phenol red free medium (Gibco) in 10% FBS (Hyclone), supplemented with bovine insulin and non-essential amino acid (Sigma). The cells were initially treated with 4-hydoxyltamoxifen (10⁻⁸ M) and let stand at 37°C for 24 hours. Following this incubation with tamoxifen, the cells were treated with compounds at various concentrations.

Compounds to be tested in the agonist mode were added to the culture media at varying concentrations. Compounds to be treated in the antagonist mode were prepared similarly, and 10 nM 17β-estradiol was also added to the culture media. The cells were incubated for 24 hours at 37°C. Following this incubation, 0.1 µCi of ¹⁴C-thymidine (56mCi/mmol, Amersham) was added to the culture media and the cells were incubated for an additional 24 hours at 37°C. The cells were then washed twice with Hank's buffered salt solution (HBSS) (Gibco) and counted with a scintillation counter. The increase in the ¹⁴C-thymidine in the compound treated cells relative to the vehicle control cells were reported as percent increase in cell proliferation.

### Example 21

### Alkaline Phosphatase Assay in Human Endometrial lshikawa Cells

This assay was run according to the procedure described by Albert et a., Cancer Res, (9910), 50(11), 330-6-10, with minor modification.

Ishikawa cells (from ATCC) were maintained in DMEM/F12 (1:1) phenol red free medium (Gibco) supplemented with 10% calf serum (Hyclone). 24 hours prior to testing, the medium was changed to DMEM/F12 (1:1) phenol red free containing 2% calf serum.

Compounds to be tested in the agonist mode were added to the culture media at varying concentrations. Compounds to be treated in the antagonist mode were prepared similarly, and 10 nM 17β-estradiol was also added to the culture media. The cells were then incubated at 37°C for 3 days. On the fourth day, the media was remove, 1 volume of 1X Dilution Buffer (Clontech) was added to the well followed by addition of 1 volume of Assay Buffer (Clontech). The cells were then incubated at room temperature for 5 minutes. 1 volume of freshly prepared Chemiluminescence Buffer (1 volume of chemiluminescent substrate (CSPD) in 19 volume Chemiluminescent Enhancer with final concentration of CSPD at 1.25 mM; Sigma Chemical Co.) was added. The cells were incubated at room temperature for 10 minutes and then quantified on a luminometer. The increase of chemiluminescence over vehicle control was used to calculate the increase in alkaline phosphatase activity.

Representative compound of the present invention were tested according to the procedure described in Examples 20 and 21 above, with results as listed in Table. 4.

**Table 4**

| **ID No.** | **MCF7 (Breast) EC₅₀ (nM)** | **Ishikawa (Endometrial) EC₅₀ (nM)** |
|---|---|---|
| 3 | 240 | 1.9 |
| 4 | 280 | 1.8 |
| 6 | 2.9 | 0.21 |
| 8 | 11 | 0.44 |
| 13 | 15 | 1.2 |

### Example 22

As a specific embodiment of an oral composition, 100 mg of the Compound #6, prepared as in Example 10 is formulated with sufficient finely divided lactose to provide a total amount of 580 to 590 mg to fill a size O hard gel capsule.

While the foregoing specification teaches the principles of the present invention, with examples provided for the purpose of illustration, it will be understood that the practice of the invention encompasses all of the usual variations, adaptations and/or modifications as come within the scope of the following claims and their equivalents.

## Claims

1. A compound of formula (I) 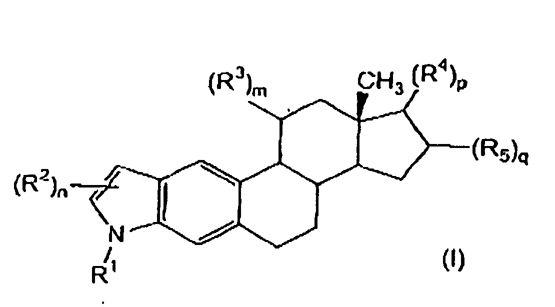 wherein
R¹ is selected from the group consisting of hydrogen, hydroxy, A, -()-A, C(O)-A and -SO₂-A;
n is an integer from 0 to 2;
each R² is independently selected from the group consisting of hydroxy, carboxy, halogen, -A, -O-A, -C(O)-A, -C(O)O-A, amino, (C1-C8)alkylamino, di(C1-C₈)alkylamino, cyano, aminocarbonyl, (C1-C8)alkylaminocarbonyl, di(C1-C8)alkylaminocarbonyl. -SH, -S-A, -SO-A, -SO₂-A, -SO₂-NH₂, -SO₂-NH(C1-C8 alkyl) and -SO₂-N(C1-C8 alkyl)₂;
m is an integer from 0 to 2;
each R³ is independently selected from the group consisting of -A, -O-A, -S-A, -NH-A, -N(A)₂ and -C(O)-A;
p is an integer from 1 to 2;
each R⁴ is independently selected from the group consisting of hydroxy, carboxy, cyano, -A, (C2-C8)alkenyl, -(C2-C8)alkynyl-A, (C2-C8)alkynyl, -(C2-C8)alkynyl-A, -O-A, -NH₂, NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H and -OC(O)-A-,
alternatively, when p is 2, two R⁴ groups may be taken together as oxo or =N(OH);
q is an integer from 0 to 2;
each R⁵ is independently selected from the group consisting of hydroxy, carboxy, halogen, (C1-C8)alkyl, (C1-C8) alkoxy, (C3-C8)cycloalkyl and -C(O)-A;
wherein the (C1-C8)alkyl group is optionally substituted with one or more substituents independently selected from halogen, hydroxy, carboxy or (C1-C8) alkoxy;
wherein each A is independently selected from the group consisting of (C1-C8)alkyl, aryl, aralkyl, (C3-C8)cycloalkyl, heteroaryl and heterocycloalkyl; wherein the aryl, aralkyl, (C3-C8)cycloalkyl, heteroaryl or heterocycloalkyl group is optionally substituted with one or more substituents independently selected from halogen, hydroxy, carboxy, (C1-C4)alkyl, (C1-C8) alkoxy, nitro, cyano, amino, (C1-C4)alkylamino or di ((C1-C4)alkyl) amino; wherein a heteroaryl is a five or six membered monocyclic aromatic ring structure containing at least one heteroatom selected from the group consisting of O, N and S or a nine of ten membered bicyclic aromatic ring structure containing at least one heteroatom selected from the group consisting of O, N and S, a heterocycloalkyl is a five to seven membered monocyclic, saturated or partially unsaturated ring structure containing at least one heteroatom selected from the group consisting of O, N and S or a nine to ten membered saturated, partially unsaturated or partially aromatic bicyclic ring system containing at least one heteroatom selected from the group consisting of O, N and S and an aryl is a carbocyclic aromatic group;
or a pharmaceutical acceptable salt thereof.

2. A compound as in Claim I wherein
R¹ is selected from the group consisting of hydrogen, hydroxy, A, -O-A, C(O)-A and -SO₂-A;
n is an integer from 0 to 1;
each R² is independently selected from the group consisting of carboxy, halogen, -A, -C(O)-A, -C(O)O-A, cyano, -S-A, -SO-A, -SO₂-A, -S0₂-NH₂, -SO₂-NH(C1-C8 alkyl) and -SO₂-N(C1-C8 alkyl)₂;
m is an integer from 0 to 1;
each R³ is independently selected from the group consisting of -A, -0-A, -S-A, - NH-A and -C(O)-A;
p is an integer from 1 to 2;
R⁴ is selected from the group consisting of hydroxy, -NH₂, -NH(A). -N(A)2, -C(O)NH₂, -C(O)-NH(A), -SO₂-NH₂, -SO₂-NH(A) and -OC(O)-A, when the R⁴ is in a β-orientation;
R⁴ is selected from the group consisting of hydroxy, carboxy, cyano, -A. (C2-C8)alkenyl, -(C2-C8)alkynyl-A, (C2-C8)alkynyl, -(C2-C8)alkynyl-A, -O-A, -NH₂, -NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H and -OC(O)-A, when the R⁴ is in an a-orientation;
alternatively, when p is 2, two R⁴ groups may be taken together as oxo or =N(OH);
q is an integer from 0 to 1;
R⁵ is selected from the group consisting of carboxy, halogen, (C1-C4)alkyl, and -C(O)-A; wherein the alkyl group is optionally substituted with one to two substituents independently selected from halogen, hydroxy, carboxy or (C1-C8) alkoxy:
wherein each A is independently selected from the group consisting of (Cl-C8)alkyl, aryl, aralkyl, (C3-C8)cycloalkyl, heteroaryl and heterocycloalkyl; wherein the aryl, aralkyl, (C3-C8)cycloalkyl, heteroaryl or heterocycloalkyl group is optionally substituted with one to two substituents independently selected from halogen, hydroxy, carboxy, (C1-C4)alkyl, (C1-C8) alkoxy, nitro, cyano, amino, (C1-C4)alkylamino or di((C1-C4)alkyl)amino;
or a pharmaceutically acceptable salt thereof.

3. A compound as in Claim 2 wherein
R¹ is selected from the group consisting of hydrogen and -SO₂-(C1-C8 alkyl);
n is 0;
m is 0;
p is an integer from 1 to 2;
R⁴ is selected from the group consisting of hydroxy and -O-C(O)-(C1-C8 alkyl); wherein the (C1-C8)alkyl portion of the -O-C(O)-(C1-C8 alkyl) group is optionally substituted with a carboxy group;
alternatively when p is 2, two R⁴ groups are taken together as oxo;
q is 0;
or a pharmaceutical acceptable salt thereof.

4. A compound as in Claim 3
wherein R¹ is selected from the group consisting of hydrogen and -SO₂-CH₃;
n is 0;
m is 0;
p is an integer from 1 to 2;
R⁴ is selected from the group consisting of hydroxy and -O-C(O)-n-butyl and -O-C(O)-CH₂CH₂CH₂CH₂CO₂H;
alternatively when p is 2, two R⁴ groups are taken together as oxo;
q is 0;
or a pharmaceutical acceptable salt thereof.

5. A compound of the formula (II) 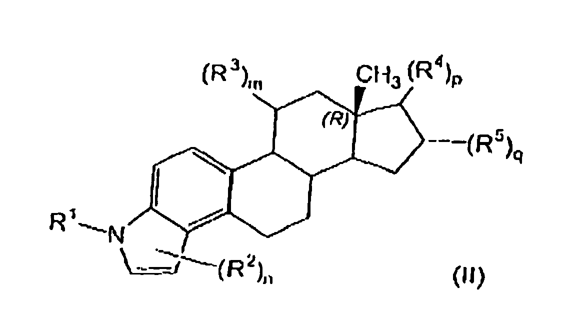 wherein
R¹ is selected from the group consisting of hydrogen, hydroxy, A, -O-A, C(O)-A and -SO₂-A;
n is an integer from () to 2;
each R₂ is independently selected from the group consisting of hydroxy, carboxy, halogen, -A, -O-A, -C(O)-A, -C(O)O-A, amino, (C1-C8)alkylamino, di(C1-C8)alkylamino, cyano, aminocarbonyl, (C1-C8)alkylaminocarbonyl, di(C1-C8)alkylaminocarbonyl, -SH, -S-A, -SO-A, -SO₂-A, -SO₂-NH₂, -SO₂-NH(C1-C8 alkyl) and -SO₂-N(C1-C8 alkyl)₂;
m is an integer from 0 to 2;
each R³ is independently selected from the group consisting of -A, -O-A, -S-A, -NH-A, -N(A)₂ and -C(O)-A;
p is an integer from 1 to 2;
each R⁴ is independently selected from the group consisting of hydroxy, carboxy, cyano, -A, (C2-C8)alkenyl, -(C2-C8)alkynyl-A, (C2-C8)alkynyl, -(C2-C8)alkynyl-A, -O-A, -NH₂, NH(A). -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H and -OC(O)-A;
alternatively, when p is 2, two R⁴ groups may be taken together as oxo or -N(OH);
q is an integer from 0 to 2;
each R⁵ is independently selected from the group consisting of hydroxy, carboxy, halogen, (C1-C8)alkyl, (C1-C8) alkoxy, (C3-C8)cycloalkyl and -C(O)-A; wherein the (C1-C8)alkyl group is optionally substituted with one or more substituents independently selected from halogen, hydroxy, carboxy or (C1-C8) alkoxy;
wherein each A is independently selected from the group, consisting of (Cl-C8)alkyl, aryl, aralkyl, (C3-C8)cycloalkyl, heteroaryl and heterocycloalkyl; wherein the aryl, aralkyl, (C3-C8)cycloalkyl, heteroaryl or heterocycloalkyl group is optionally substituted with one or more substituents independently selected from halogen, hydroxy, carboxy, (C1-C4)alkyl, (C1-C8) alkoxy, nitro, cyano, amino, (C1-C4)alkylamino or di((C1-C4)alkyl)amino; wherein a heteroaryl is a five or six membered monocyclic aromatic ring structure containing at least one heteroatom selected from the group consisting of O, N and S or a nine of ten membered bicyclic aromatic ring structure containing at least one heteroatom selected from the group consisting of O, N and S, a heterocycloalkyl is a five to seven membered monocyclic, saturated or partially unsaturated ring structure containing at least one heteroatom selected from the group consisting of O, N and S or a nine to ten membered saturated, partially unsaturated or partially aromatic bicyclic ring system containing at least one heteroatom selected from the group consisting of O, N and S and an aryl is a carbocyclic aromatic group;
or a pharmaceutically acceptable salt thereof.

6. A compound as in Claim 5
wherein R¹ is selected from the group consisting of hydrogen, hydroxy, A, -O-A, C(O)-A and -SO₂-A;
n is an integer from 0 to 1;
each R² is independently selected from the group consisting of carboxy, halogen, -A, -C(O)-A, -C(O)O-A, cyano, -SO-A, -SO₂-A, -SO₂-NH₂, -SO₂-NH(C1-C8 alkyl) and -SO₂-N(C1-C8 alkyl)₂;
m is an integer from 0 to 1;
each R³ is independently selected from the group consisting of -A, -O-A, -S-A, -NH-A and -C(O)-A;
p is an integer from 1 to 2;
R⁴ is selected from the group consisting of hydroxy, -NH₂, -NH(A), -N(A)₂, -C(O)NH₂, -C(O)-NH(A), -SO₂-NH₂, -SO₂-NH(A) and -OC(O)-A, when the R⁴ is in a β-orientation;
R⁴ is selected from the group consisting of hydroxy, carboxy, cyano, -A, (C2-C8)alkenyl, -(C2-C8)alkynyl-A, (C2-C8)alkynyl, -(C2-C8)alkynyl-A. -O-A, -NH₂, -NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -Nli-SO₂-A, -C(O)O-A, -OC(O)H and -OC(O)-A, when the R⁴ is in an α-orientation;
alternatively, when p is 2, two R⁴ groups may be taken together as oxo or =N(OH);
q is an integer from 0 to 1;
R⁵ is selected from the group consisting of carboxy, halogen, (C1-C4)alkyl, and-C(O)-A; wherein the alkyl group is optionally substituted with one to two substituents independently selected from halogen, hydroxy, carboxy or (C1-C8) alkoxy,
wherein each A is independently selected from the group consisting of (C1-C8)alkyl, aryl, aralkyl, (C3-C8)cyclalkyl, heteroaryl and heterocycloalkyl; wherein the aryl, aralkyl, (C3-C8)cycloalkyl, heteroaryl or heterocycloalkyl group is optionally substituted with one to two substituents independently selected from halogen, hydroxy, carboxy, (C1-C4)alkyl, (C1-C8) alkoxy, nitro, cyano, amino, (C1-C4)alkylamino or di((C1-C4)alkyl)amino;
or a pharmaceutically acceptable salt thereof.

7. A compound as in Claim 6 wherein
R¹ is selected from the group consisting of hydrogen and -SO₂-(C1-C8 alkyl);
n is an integer from 0 to 1;
R² is selected from the group consisting of -S-(C 1-C8 alkyl);
m is 0;
p is an integer from 1 to 2;
R⁴ is selected from the group consisting of hydroxy, (C2-C8)alkynyl and -O-C(O)-(C1-C8 alkyl);
alternatively, when p is 2, two R⁴ groups are taken together as oxo:
q is 0;
or a pharmaceutically acceptable salt thereof

8. A compound as in Claim 7 wherein
R¹ is selected from the group consisting of hydrogen and -SO₂-CH₃;
n is an integer from 0 to 1;
R² is -S-CH₃;
m is 0;
p is an integer from 1 to 2;
R⁴ is selected from the group consisting of hydroxy, ethynyl and -OC(O)-n-butyl;
alternatively, when p is 2, two R⁴ groups are taken together as oxo;
q is 0;
or a pharmaceutically acceptable salt thereof.

9. A pharmaceutical composition comprising a pharmaceutically acceptable carrier and a compound of Claim 1 or Claim 5.

10. A pharmaceutical composition made by mixing a compound of Claim 1 or Claim 5 and a pharmaceutically acceptable carrier.

11. A process for making a pharmaceutical composition comprising mixing a compound of Claim 1 or Claim 5 and a pharmaceutically acceptable carrier.

12. The compound of Claim 1 or Claim 5 for use in therapy.

13. The use of a compound of Claim 1 or Claim 5 for the manufacture of a medicament for the treatment of a disorder mediated by an estrogen receptor.

14. The use of Claim 13, wherein the disorder mediated by an estrogen receptor is selected from the group consisting of hot flashes, vaginal dryness, osteopenia, osteoporosis, hyperlipidemia, loss of cognitive function, degenerative brain diseases, cardiovascular diseases, cerebrovascular diseases, cancer of the breast tissue, hyperplasia of the breast tissue, cancer or the endometrium, hyperplasia of the endometrium, cancer of the cervix, hyperplasia of the cervix, cancer of the prostate, benign prostate hyperplasia, endometriosis, uterine fibroids, osteoarthritis and contraception.

15. The use of Claim 14, wherein the disorder mediated by an estrogen receptor is selected from the group consisting of osteoporosis, hot flashes, vaginal dryness, breast cancer and endometriosis.

16. The pharmaceutical composition of Claim 10 for use in therapy.

17. The use of a pharmaceutical composition of Claim 10 for the manufacture of a medicament for the treatment of a disorder mediated by an estrogen receptor.

## Patentansprüche

1. Verbindung von Formel (I) 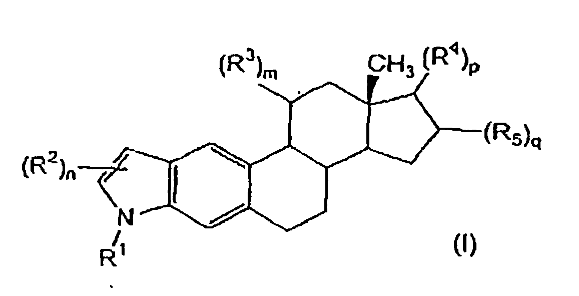 worin
R¹ ausgewählt ist aus der Gruppe, bestehend aus Wasserstoff, Hydroxy, A, -O-A, C(O)-A und -SO₂-A;
n eine ganze Zahl von 0 bis 2 ist;
R² jeweils unabhängig ausgewählt ist aus der Gruppe, bestehend aus Hydroxy, Carboxy, Halogen, -A, -O-A, -C(O)-A, -C(O)O-A, Amino, (C1-C8)-Alkylamino, Di-(C1-C8)-alkylamino, Cyano, Aminocarbonyl, (C1-C8)-Alkylaminocarbonyl, Di-(C1-C8)-alkylaminocarbonyl, -SH, -S-A, -SO-A, -SO₂-A, -SO₂-NH₂, -SO₂-NH(C1-C8-Alkyl) und -SO₂-N(C1-C8-Alkyl)₂;
m eine ganze Zahl von 0 bis 2 ist;
R³ jeweils unabhängig ausgewählt ist aus der Gruppe, bestehend aus -A, -O-A, -S-A, -NH-A, -N(A)₂ und -C(O)-A;
p eine ganze Zahl von 1 bis 2 ist;
R⁴ jeweils unabhängig ausgewählt ist aus der Gruppe, bestehend Hydroxy, Carboxy, Cyano, -A, (C2-C8)-Alkenyl, -(C2-C8)-Alkinyl-A, (C2-C8)-Alkinyl, -(C2-C8)-Alkinyl-A, -O-A, -NH₂, NH(A), -N(A)₂, -N(A)-C(O)-A, -NH-C(O)-A, -C(O)-N(A)₂, -C(O)-NH₂, C(O)-NH-A, -SO₂N(A)₂, -SO₂-NH(A), -SO₂-NH₂, -N(A)-SO₂-A, -NH-SO₂-A, -C(O)O-A, -OC(O)H und -OC(O)-A;
alternativ, wenn p 2 ist, zwei R⁴-Gruppen als Oxo- oder =N(OH) zusammengenommen sein können;
q eine ganze Zahl von 0 bis 2 ist;
R⁵ jeweils unabhängig ausgewählt ist aus der Gruppe, bestehend aus Hydroxy, Carboxy, Halogen, (C1-C8)-Alkyl, (C1-C8)-Alkoxy, (C3-C8)-Cycloalkyl und -C(O)-A;
wobei die (C1-C8)-Alkylgruppe fakultativ mit einem oder mehreren Substituenten substituiert ist, die unabhängig ausgewählt sind aus Halogen, Hydroxy, Carboxy oder (C1-C8)-Alkoxy;
wobei A jeweils unabhängig ausgewählt ist aus der Gruppe, bestehend aus (C1-C8)-Alkyl, Aryl, Aralkyl, (C3-C8)-Cycloalkyl, Heteroaryl und Heterocycloalkyl; wobei die Aryl-, Aralkyl-, (C3-C8)-Cycloalkyl-, Heteroaryl- oder Heterocycloalkylgruppe fakultativ mit einem oder mehreren Substituenten substituiert ist, die unabhängig ausgewählt sind aus Halogen, Hydroxy, Carboxy, (C1-C4)-Alkyl, (C1-C8)-Alkoxy, Nitro, Cyano, Amino, (C1-C4)-Alkylamino oder Di-((C1-C4)-alkyl)-amino; wobei ein Heteroaryl eine fünf- oder sechsgliedrige monocyclische aromatische Ringstruktur ist, die wenigstens ein Heteroatom enthält, das ausgewählt ist aus der Gruppe, bestehend aus 0, N und S, oder eine neun- oder zehngliedrige bicyclische aromatische Ringstruktur, die wenigstens ein Heteroatom enthält, das ausgewählt ist aus der Gruppe, bestehend aus O, N und S, ein Heterocycloalkyl eine fünf- bis siebengliedrige monocyclische, gesättigte oder teilweise ungesättigte Ringstruktur ist, die wenigstens ein Heteroatom enthält, das ausgewählt ist aus der Gruppe, bestehend aus O, N und S, oder ein neun- bis zehngliedriges gesättigtes, teilweise ungesättigtes oder teilweise aromatisches bicyclisches Ringsystem, das wenigstens ein Heteroatom enthält, das ausgewählt ist aus der Gruppe, bestehend aus O, N und S, und ein Aryl eine carbocyclische aromatische Gruppe ist;
oder ein pharmazeutisch annehmbares Salz davon.

2. Verbindung nach Anspruch 1, **dadurch gekennzeichnet, daß**
R¹ ausgewählt ist aus der Gruppe, bestehend aus Wasserstoff, Hydroxy, A, -O-A, C(O)-A und -SO₂-A;
n eine ganze Zahl von 0 bis 1 ist;
R² jeweils unabhängig ausgewählt ist aus der Gruppe, bestehend aus Carboxy, Halogen, -A, -C(O)-A, -C(O)O-A, Cyano, -S-A, -SO-A, -SO₂-A, -SO₂-NH₂, -SO₂-NH(C1-C8-Alkyl) und -SO₂-N(C1-C8-Alkyl)₂;
m eine ganze Zahl von 0 bis 1 ist;
R³ jeweils unabhängig ausgewählt ist aus der Gruppe, bestehend aus -A, -O-A, -S-A, -NH-A und -C(O)-A;
p eine ganze Zahl von 1 bis 2 ist;
R⁴ ausgewählt ist aus der Gruppe, bestehend aus Hydroxy, -NH₂, -NH(A), -N(A)₂, -C(O)NH₂, -C(O)-NH(A), -SO₂-NH₂, -SO₂-NH(A) und -OC(O)-A, wenn R⁴ in einer β-Orientierung ist;
R⁴ ausgewählt ist aus der Gruppe, bestehend aus Hydroxy, Carboxy, Cyano, -A, (C2-C8)-Alkenyl, -(C2-C8)-Alkinyl-A, (C2-C8)-Alkinyl, -(C2-C8)-Alkinyl-A, -O-A, -NH₂, -NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H und -OC(O)-A, wenn R⁴ in einer α-Orientierung ist;
alternativ, wenn p 2 ist, zwei R⁴ Gruppen als Oxo oder =N(OH) zusammengenommen sein können;
q eine ganze Zahl von 0 bis 1 ist;
R⁵ ausgewählt ist aus der Gruppe, bestehend aus Carboxy, Halogen, (C1-C4)-Alkyl und -C(O)-A; wobei die Alkylgruppe fakultativ mit einem bis zwei Substituenten substituiert ist, die unabhängig ausgewählt sind aus Halogen, Hydroxy, Carboxy oder (C1-C8)-Alkoxy;
wobei A jeweils unabhängig ausgewählt ist aus der Gruppe, bestehend aus (C1-C8)-Alkyl, Aryl, Aralkyl, (C3-C8)-Cycloalkyl, Heteroaryl und Heterocycloalkyl; wobei die Aryl-, Aralkyl-, (C3-C8)-Cycloalkyl-, Heteroaryl- oder Hetercycloalkylgruppe fakultativ mit einem bis zwei Substituenten substituiert ist, die unabhängig ausgewählt sind aus Halogen, Hydroxy, Carboxy, (C1-C4)-Alkyl, (C1-C8)-Alkoxy, Nitro, Cyano, Amino, (C1-C4)-Alkylamino oder Di-((C1-C4)-alkyl)-amino;
oder ein pharmazeutisch annehmbares Salz davon.

3. Verbindung nach Anspruch 2, **dadurch gekennzeichnet, daß**
R¹ ausgewählt ist aus der Gruppe, bestehend aus Wasserstoff und -SO₂-(C1-C8-Alkyl);
n 0 ist;
m 0 ist;
p eine ganze Zahl von 1 bis 2 ist;
R⁴ ausgewählt ist aus der Gruppe, bestehend aus Hydroxy und -O-C(O)-(C1-C8-Alkyl); wobei der (C1-C8)-Alkylteil der -O-C(O)-(Cl-C8-Alkyl)-Gruppe fakultativ mit einer Carboxygruppe substituiert ist;
alternativ, wenn p 2 ist, zwei R⁴-Gruppen als Oxo zusammengenommen sind;
q 0 ist;
oder ein pharmazeutisch annehmbares Salz davon.

4. Verbindung nach Anspruch 3, **dadurch gekennzeichnet, daß**
R¹ ausgewählt ist aus der Gruppe, bestehend aus Wasserstoff und -SO₂-CH₃;
n 0 ist;
m 0 ist;
p eine ganze Zahl von 1 bis 2 ist;
R⁴ ausgewählt ist aus der Gruppe, bestehend aus Hydroxy und -O-C(O)-n-Butyl und -O-C(0)-CH₂CH₂CH₂CH₂-CO₂H;
alternativ, wenn p 2, zwei R⁴-Gruppen als Oxo zusammengenommen sind;
q 0 ist;
oder ein pharmazeutisch annehmbares Salz davon.

5. Verbindung der Formel (II) 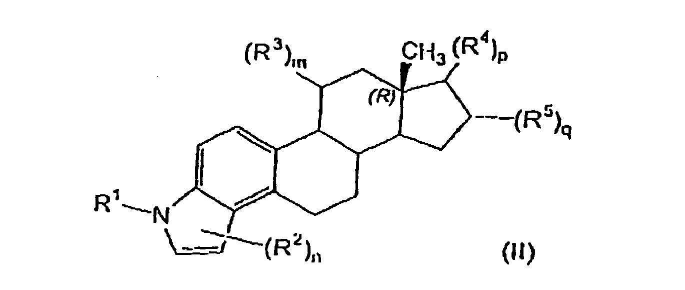 worin
R¹ ausgewählt ist aus der Gruppe, bestehend aus Wasserstoff, Hydroxy, A, -O-A, C(O)-A und -SO₂-A;
n eine ganze Zahl von 0 bis 2 ist;
R² jeweils unabhängig ausgewählt ist aus der Gruppe, bestehend aus Hydroxy, Carboxy, Halogen, -A, -O-A, -C(O)-A, -C(O)O-A, Amino, (C1-C8)-Alkylamino, Di-(C1-C8)-alkylamino, Cyano, Aminocarbonyl, (C1-C8)-Alkylaminocarbonyl, Di-(C1-C8)-alkylaminocarbonyl, -SH, -S-A, -SO-A, -SO₂-A, -SO₂-NH₂, -SO₂-NH(C1-C8-Alkyl) und -SO₂-N(C1-C8-Alkyl)₂;
m eine ganze Zahl von 0 bis 2 ist;
R³ jeweils unabhängig ausgewählt ist aus der Gruppe, bestehend aus -A, -O-A, -S-A, -NH-A, -N(A)₂ und -C(O)-A;
p eine ganze Zahl von 1 bis 2 ist;
R⁴ jeweils unabhängig ausgewählt ist aus der Gruppe, bestehend aus Hydroxy, Carboxy, Cyano, -A, (C2-C8)-Alkenyl, -(C2-C8)-Alkinyl-A, (C2-C8)-Alkinyl, (C2-C8)-Alkinyl-A, -O-A, -NH₂, NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂NH₂, -N(A)-SO₂-A, -NH-SO₂-A, -C(O)O-A, -OC(O)H und -OC(O)-A;
alternativ, wenn p 2 ist, zwei R⁴-Gruppen als Oxo- oder =N(OH) zusammengenommen sein können;
q eine ganze Zahl von 0 bis 2 ist;
R⁵ jeweils unabhängig ausgewählt ist aus der Gruppe, bestehend aus Hydroxy, Carboxy, Halogen, (C1-C8)-Alkyl, (C1-C8)-Alkoxy, (C3-C8)-Cycloalkyl und -C(O)-A; wobei die (C1-C8)-Alkylgruppe fakultativ mit einem oder mehreren Substituenten substituiert ist, die unabhängig ausgewählt sind aus Halogen, Hydroxy, Carboxy oder (C1-C8)-Alkoxy;
wobei A jeweils unabhängig ausgewählt ist aus der Gruppe, bestehend aus (C1-C8)-Alkyl, Aryl, Aralkyl, (C3-C8)-Cycloalkyl, Heteroaryl und Heterocycloalkyl; wobei die Aryl-, Aralkyl-, (C3-C8)-Cycloalkyl-, Heteroaryl- oder Heterocycloalkylgruppe fakultativ mit einem oder mehreren Substituenten substituiert ist, die unabhängig ausgewählt sind aus Halogen, Hydroxy, Carboxy, (C1-C4)-Alkyl, (C1-C8)-Alkoxy, Nitro, Cyano, Amino, (C1-C4)-Alkylamino oder Di-((C 1-C4)-alkyl)-amino; wobei ein Heteroaryl eine fünf- oder sechsgliedrige monocyclische aromatische Ringstruktur ist, die wenigstens ein Heteroatom enthält, das ausgewählt ist aus der Gruppe, bestehend aus O, N und S, oder eine neun- oder zehngliedrige bicyclische aromatische Ringstruktur, die wenigstens ein Heteroatom enthält, das ausgewählt ist aus der Gruppe, bestehend aus O, N und S, ein Heterocycloalkyl eine fünf- bis siebengliedrige monocyclische, gesättigte oder teilweise ungesättigte Ringstruktur ist, die wenigstens ein Heteroatom enthält, das ausgewählt ist aus der Gruppe, bestehend aus O, N und S, oder ein neun- bis zehngliedriges gesättigtes, teilweise ungesättigtes oder teilweise aromatisches bicyclisches Ringsystem, das wenigstens ein Heteroatom enthält, das ausgewählt ist aus der Gruppe, bestehend aus O, N und S, und ein Aryl eine carbocyclische aromatische Gruppe ist;
oder ein pharmazeutisch annehmbares Salz davon.

6. Verbindung nach Anspruch 5, **dadurch gekennzeichnet, daß**
R¹ ausgewählt ist aus der Gruppe, bestehend aus Wasserstoff, Hydroxy, A, -O-A, C(O)-A und -SO₂-A;
n eine ganze Zahl von 0 bis 1 ist;
R² jeweils unabhängig ausgewählt ist aus der Gruppe, bestehend aus Carboxy, Halogen, -A, -C(O)-A, -C(O)O-A, Cyano, -SO-A, -SO₂-A, -SO₂-NH₂, -SO₂-NH(C1-C8-Alkyl) und -SO₂-N(C1-C8-Alkyl)₂;
m eine ganze Zahl von 0 bis 1 ist;
R³ jeweils unabhängig ausgewählt ist aus der Gruppe, bestehend aus -A, -O-A, -S-A, -NH-A und -C(O)-A;
p eine ganze Zahl von 1 bis 2 ist;
R⁴ ausgewählt ist aus der Gruppe, bestehend aus Hydroxy, -NH₂, -NH(A), -N(A)₂, -C(O)NH₂, -C(O)-NH(A), -SO₂-NH₂, -SO₂-NH(A) und -OC(O)-A, wenn R⁴ in einer β-Orientierung ist;
R⁴ ausgewählt ist aus der Gruppe, bestehend aus Hydroxy, Carboxy, Cyano, -A, (C2-C8)-Alkenyl, -(C2-C8)-Alkinyl-A, (C2-C8)-Alkinyl, -(C2-C8)-Alkinyl-A, -O-A, -NH₂, -NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H und -OC(O)-A, wenn R⁴ in einer α-Orientierung ist;
alternativ, wenn p 2 ist, zwei R⁴ Gruppen als Oxo oder =N(OH) zusammengenommen sein können;
q eine ganze Zahl von 0 bis 1 ist;
R⁵ ausgewählt ist aus der Gruppe, bestehend aus Carboxy, Halogen, (C1-C4)-Alkyl und -C(O)-A; wobei die Alkylgruppe fakultativ mit einem bis zwei Substituenten substituiert ist, die unabhängig ausgewählt sind aus Halogen, Hydroxy, Carboxy oder (C1-C8)-Alkoxy;
wobei A jeweils unabhängig ausgewählt ist aus der Gruppe, bestehend aus (C1-C8)-Alkyl, Aryl, Aralkyl, (C3-C8)-Cycloalkyl, Heteroaryl und Heterocycloalkyl; wobei die Aryl-, Aralkyl-, (C3-C8)-Cycloalkyl-, Heteroaryl- oder Hetercycloalkylgruppe fakultativ mit einem bis zwei Substituenten substituiert ist, die unabhängig ausgewählt sind aus Halogen, Hydroxy, Carboxy, (C1-C4)-Alkyl, (C1-C8)-Alkoxy, Nitro, Cyano, Amino, (C1-C4)-Alkylamino oder Di-((C1-C4)-alkyl)-amino;
oder ein pharmazeutisch annehmbares Salz davon.

7. Verbindung nach Anspruch 6, **dadurch gekennzeichnet, daß**
R¹ ausgewählt ist aus der Gruppe, bestehend aus Wasserstoff und -SO₂-(C₁-C₈-Alkyl);
n eine ganze Zahl von 0 bis 1 ist;
R² ausgewählt ist aus der Gruppe, bestehend aus -S-(C1-C8-Alkyl);
m 0 ist;
p eine ganze Zahl von 1 bis 2 ist;
R⁴ ausgewählt ist aus der Gruppe, bestehend aus Hydroxy, (C2-C8)-Alkinyl und -O-C(O)-(C₁-C₈-Alkyl);
alternativ, wenn p 2 ist, zwei R⁴-Gruppen als Oxo zusammengenommen sind;
q 0 ist;
oder ein pharmazeutisch annehmbares Salz davon.

8. Verbindung nach Anspruch 7, **dadurch gekennzeichnet, daß**
R¹ ausgewählt ist aus der Gruppe, bestehend aus Wasserstoff und -SO₂-CH₃;
n eine ganze Zahl von 0 bis 1 ist;
R² -S-CH₃ ist;
m 0 ist;
p eine ganze Zahl von 1 bis 2 ist;
R⁴ ausgewählt ist aus der Gruppe, bestehend aus Hydroxy, Ethinyl und -O-C(O)-n-Butyl;
alternativ, wenn p 2, zwei R⁴-Gruppen als Oxo zusammengenommen sind;
q 0 ist;
oder ein pharmazeutisch annehmbares Salz davon.

9. Pharmazeutische Zusammensetzung, die einen pharmazeutisch annehmbaren Trägerstoff und eine Verbindung nach Anspruch 1 oder Anspruch 5 umfasst.

10. Pharmazeutische Zusammensetzung, hergestellt durch Vermischen einer Verbindung nach Anspruch 1 oder Anspruch 5 und eines pharmazeutisch annehmbaren Trägerstoffes.

11. Verfahren zur Herstellung einer pharmazeutischen Zusammensetzung, welches das Vermischen einer Verbindung nach Anspruch 1 oder Anspruch 5 und eines pharmazeutisch annehmbaren Trägerstoffes umfasst.

12. Verbindung nach Anspruch 1 oder Anspruch 5 zur Verwendung in der Therapie.

13. Verwendung einer Verbindung nach Anspruch 1 oder Anspruch 5 zur Herstellung eines Arzneimittels zur Behandlung einer Störung, die durch einen Estrogen-Rezeptor vermittelt ist.

14. Verwendung nach Anspruch 13, **dadurch gekennzeichnet, daß** die Störung, die durch einen Estrogen-Rezeptor vermittelt ist, ausgewählt ist aus der Gruppe, bestehend aus Hitzewallungen, vaginaler Trockenheit, Osteopenie, Osteoporose, Hyperlipidämie, Verlust kognitiver Funktion, degenerativen Hirnerkrankungen, kardiovaskulären Erkrankungen, zerebrovaskulären Erkrankungen, Krebs des Brustgewebes, Hyperplasie des Brustgewebes, Krebs der Gebärmutterschleimhaut, Hyperplasie der Gebärmutterschleimhaut, Krebs des Gebärmutterhalses, Hyperplasie des Gebärmutterhalses, Krebs der Prostata, benigner Prostatahyperplasie, Endometriose, Gebärmutterfibroiden, Osteoarthritis und Empfängnisverhütung.

15. Verwendung nach Anspruch 14, **dadurch gekennzeichnet, daß** die Störung, die durch einen Estrogen-Rezeptor vermittelt ist, ausgewählt ist aus der Gruppe, bestehend aus Osteoporose, Hitzewallungen, vaginaler Trockenheit, Brustkrebs und Endometriose.

16. Pharmazeutische Zusammensetzung nach Anspruch 10 zur Verwendung in der Therapie.

17. Verwendung einer pharmazeutischen Zusammensetzung nach Anspruch 10 zur Herstellung eines Arzneimittels zur Behandlung einer Störung, die durch einen Estrogen-Rezeptor vermittelt ist.

## Revendications

1. Composé de formule (I) : 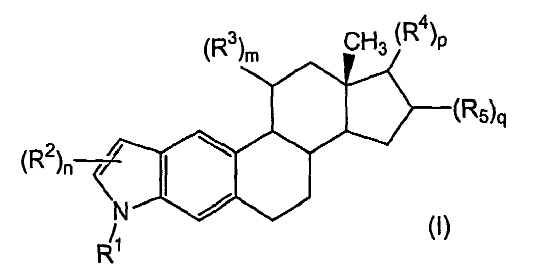 dans laquelle :
R¹ est choisi dans le groupe consistant en un atome d'hydrogène, un groupe hydroxy, -A, -O-A-, C(O)-A et -SO₂-A ;
n est un nombre entier de 0 à 2 ;
chaque R² est indépendamment choisi dans le groupe consistant en un groupe hydroxy, carboxy, un atome d'halogène, -A, -O-A, -C(O)-A, -C(O)O-A, un groupe amino, alkylamino (C₁-C₈), dialkylamino (C₁-C₈), cyano, aminocarbonyle, alkylaminocarbonyle (C₁-C₈), dialkylaminocarbonyle (C₁-C₈), -SH, -S-A, -SO-A, -SO₂-A, -SO₂-NH₂, -SO₂-NH(alkyle (C₁-C₈)) et -SO₂-N(alkyle (C₁-C₈))₂;
m est un nombre entier de 0 à 2 ;
chaque R³ est indépendamment choisi dans le groupe consistant en -A, -O-A, -S-A, -NH-A, -N(A)₂ et -C(O)-A ;
p est un nombre entier de 1 à 2 ;
chaque R⁴ est indépendamment choisi dans le groupe consistant en un groupe hydroxy, carboxy, cyano, -A, alcényle (C₂-C₈), alcényle (C₂-C₈) -A, alcynyle (C₂-C₈), alcynyle (C₂-C₈) -A, -O-A, -NH₂, NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H et -OC(O)-A ;
en variante, lorsque p est 2, deux groupes _{R}⁴ peuvent être considérés ensemble comme étant oxo
ou =N(OH) ;
q est un nombre entier de 0 à 2 ;
chaque R⁵ est indépendamment choisi dans le groupe consistant en un groupe hydroxy, carboxy, un atome d'halogène, un groupe alkyle (C₁-C₈), alcoxy (C₁-C₈), cycloalkyle (C₃-C₈) et -C(O)-A ;
dans lequel le groupe alkyle (C₁-C₈) est éventuellement substitué par un ou plusieurs substituants indépendamment choisis parmi un atome d'halogène, un groupe hydroxy, carboxy ou alcoxy (C₁-C₈) ;
dans lequel chaque A est indépendamment choisi dans le groupe consistant en un groupe alkyle (C₁-C₈), aryle, aralkyle, cycloalkyle (C₃-C₈), hétéroaryle et hétérocycloalkyle ; dans lequel le groupe aryle, aralkyle, cycloalkyle (C₃-C₈), hétéroaryle et hétérocycloalkyle est éventuellement substitué par un ou plusieurs substituants indépendamment choisis parmi un atome d'halogène, un groupe hydroxy, carboxy, alkyle (C₁-C₄), alcoxy (C₁-C₈), nitro, cyano, amino, alkylamino (C₁-C₄) ou di-(alkyle (C₁-C₄))amino ; dans lequel un groupe hétéroaryle est une structure cyclique aromatique monocyclique de cinq ou six membres, contenant au moins un hétéroatome choisi dans le groupe consistant en O, N et S ou une structure cyclique aromatique bicyclique de neuf ou dix membres, contenant au moins un hétéroatome choisi dans le groupe consistant en O, N et S, un groupe hétérocycloalkyle est une structure cyclique saturée ou partiellement insaturée, monocyclique de cinq à sept membres, contenant au moins un hétéroatome choisi dans le groupe consistant en O, N et S ou un système cyclique bicyclique saturé, partiellement insaturé ou partiellement aromatique de neuf à dix membres, contenant au moins un hétéroatome choisi dans le groupe consistant en O, N et S, et un groupe aryle est un groupe aromatique carbocyclique ;
ou sel pharmaceutiquement acceptable de celui-ci.

2. Composé selon la revendication 1, dans lequel :
R¹ est choisi dans le groupe consistant en un atome d'hydrogène, un groupe hydroxy, -A, -O-A-, -C(O)-A et -SO₂-A ;
n est un nombre entier de 0 à 1 ;
chaque R² est indépendamment choisi dans le groupe consistant en un groupe carboxy, un atome d'halogène, -A, -C(O)-A, -C(O)O-A, un groupe cyano, -S-A, -SO-A, -SO₂-A, -SO₂-NH₂, -SO₂-NH(alkyle (C₁-C₈)) et -SO₂-N(alkyle (C₁-C₈))₂ ;
m est un nombre entier de 0 à 1 ;
chaque R³ est indépendamment choisi dans le groupe consistant en -A, -O-A, -S-A, -NH-A, et -C(O)-A ;
p est un nombre entier de 1 à 2 ;
R⁴ est choisi dans le groupe consistant en un groupe hydroxy, -NH₂, NH(A), -N(A)₂, -C(O)NH₂, -C(O)-NH(A), -SO₂-NH₂, -SO₂-NH(A) et -OC(O)-A, lorsque le R⁴ est dans une orientation β ;
R⁴ est choisi dans le groupe consistant en un groupe hydroxy, carboxy, cyano, -A, alcényle (C₂-C₈), alcényle (C₂-C₈)-A, alcynyle (C₂-C₈), alcynyle (C₂-C₈)-A, -O-A, -NH₂, -NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H et -OC(O)-A, lorsque le R⁴ est dans une orientation α ;
en variante, lorsque p est 2, deux groupes R⁴ peuvent être considérés ensemble comme étant oxo ou =N(OH) ;
q est un nombre entier de 0 à 1 ;
R⁵ est choisi dans le groupe consistant en un groupe carboxy, un atome d'halogène, un groupe alkyle (C₁-C₄) et -C(O)-A ; dans lequel le groupe alkyle est éventuellement substitué par un ou plusieurs substituants indépendamment choisis parmi un atome d'halogène, un groupe hydroxy, carboxy ou alcoxy (C₁-C₈) ;
dans lequel chaque A est indépendamment choisi dans le groupe consistant en un groupe alkyle (C₁-C₈), aryle, aralkyle, cycloalkyle (C₃-C₈), hétéroaryle et hétérocycloalkyle ; dans lequel le groupe aryle, aralkyle, cycloalkyle (C₃-C₈), hétéroaryle et hétérocycloalkyle est éventuellement substitué par un ou plusieurs substituants indépendamment choisis parmi un atome d'halogène, un groupe hydroxy, carboxy, alkyle (C₁-C₄), alcoxy (C₁-C₈), nitro, cyano, amino, alkylamino (C₁-C₄) ou di-(alkyle (C₁-C₄))amino ;
ou sel pharmaceutiquement acceptable de celui-ci.

3. Composé selon la revendication 2, dans lequel :
R¹ est choisi dans le groupe consistant en un atome d' hydrogène et un groupe -SO₂-(alkyle (C₁-C₈));
n est 0 ;
m est 0 ;
p est un nombre entier de 1 à 2 ;
R⁴ est choisi dans le groupe consistant en un groupe hydroxy et un groupe -O-C(O)-(alkyle (C₁-C₈)) ; dans lequel la partie alkyle (C₁-C₈) du groupe -O-C(O)-(alkyle (C₁-C₈)) est éventuellement substituée par un groupe carboxy ;
en variante, lorsque p est 2, deux groupes R⁴ peuvent être considérés ensemble comme étant oxo ;
q est 0 ;
ou sel pharmaceutiquement acceptable de celui-ci.

4. Composé selon la revendication 3, dans lequel :
R¹ est choisi dans le groupe consistant en un atome d'hydrogène et un groupe -SO₂-CH₃ ;
n est 0 ;
m est 0 ;
p est un nombre entier de 1 à 2 ;
R⁴ est choisi dans le groupe consistant en un groupe hydroxy et un groupe -O-C(O)-n-butyle et -O-C(O)-CH₂CH₂CH₂CH₂-CO₂H ;
en variante, lorsque p est 2, deux groupes R⁴ peuvent être considérés ensemble comme étant oxo ;
q est 0 ;
ou sel pharmaceutiquement acceptable de celui-ci.

5. Composé de formule (II) : 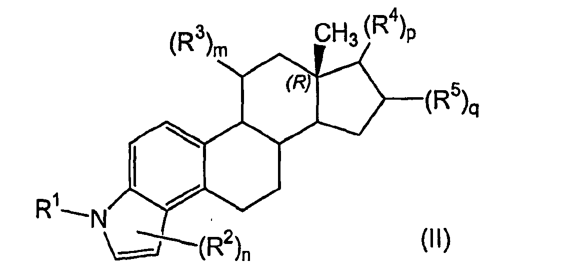 dans laquelle :
R¹ est choisi dans le groupe consistant en un atome d'hydrogène, un groupe hydroxy, -A, -O-A-, C(O)-A et -SO₂-A ;
n est un nombre entier de 0 à 2 ;
chaque R² est indépendamment choisi dans le groupe consistant en un groupe hydroxy, carboxy, un atome d'halogène, -A, -O-A, -C(O)-A, -C(O)O-A, un groupe amino, alkylamino (C₁-C₈), dialkylamino (C₁-C₈), cyano, aminocarbonyle, alkylaminocarbonyle (C₁-C₈), di-alkylaminocarbonyle (C₁-C₈), -SH, -S-A, -SO-A, -SO₂-A, -SO₂-NH₂, -SO₂-NH-(alkyle (C₁-C₈)) et -SO₂-N(alkyle (C₁-C₈))₂ ;
m est un nombre entier de 0 à 2 ;
chaque R³ est indépendamment choisi dans le groupe consistant en -A, -O-A, -S-A, -NH-A, -N(A)₂ et -C(O)-A ;
p est un nombre entier de 1 à 2 ;
chaque R⁴ est indépendamment choisi dans le groupe consistant en un groupe hydroxy, carboxy, cyano, -A, alcényle (C₂-C₈), alcényle (C₂-C₈) -A, alcynyle (C₂-C₈), alcynyle (C₂-C₈) -A, -O-A, -NH₂, -NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H et -OC(O)-A ;
en variante, lorsque p est 2, deux groupes R⁴ peuvent être considérés ensemble comme étant oxo ou =N(OH) ;
q est un nombre entier de 0 à 2 ;
chaque R⁵ est choisi dans le groupe consistant en un groupe hydroxy, carboxy, un atome d'halogène, un groupe alkyle (C₁-C₈), alcoxy (C₁-C₈), cycloalkyle (C₃-C₈) et -C(O)-A ; dans lequel le groupe alkyle (C₁-C₈) est éventuellement substitué par un ou plusieurs substituants indépendamment choisis parmi un atome d'halogène, un groupe hydroxy, carboxy ou alcoxy (C₁-C₈) ;
dans lequel chaque A est indépendamment choisi dans le groupe consistant en un groupe alkyle (C₁-C₈), aryle, aralkyle, cycloalkyle (C₃-C₈), hétéroaryle et hétérocycloalkyle ; dans lequel le groupe aryle, aralkyle, cycloalkyle (C₃-C₈), hétéroaryle et hétérocycloalkyle est éventuellement substitué par un ou plusieurs substituants indépendamment choisis parmi un atome d'halogène, un groupe hydroxy, carboxy, alkyle (C₁-C₄), alcoxy (C₁-C₈), nitro, cyano, amino, alkylamino (C₁-C₄) ou di- (alkyle (C₁-C₄)) amino ; dans lequel un groupe hétéroaryle est une structure cyclique aromatique monocyclique de cinq ou six membres, contenant au moins un hétéroatome choisi dans le groupe consistant en O, N et S ou une structure cyclique aromatique bicyclique de neuf ou dix membres, contenant au moins un hétéroatome choisi dans le groupe consistant en O, N et S, un groupe hétérocycloalkyle est une structure cyclique saturée ou partiellement insaturée, monocyclique de cinq à sept membres, contenant au moins un hétéroatome choisi dans le groupe consistant en 0, N et S ou un système cyclique bicyclique saturé, partiellement insaturé ou partiellement aromatique de neuf à dix membres, contenant au moins un hétéroatome choisi dans le groupe consistant en O, N et S, et un groupe aryle est un groupe aromatique carbocyclique ;
ou sel pharmaceutiquement acceptable de celui-ci.

6. Composé selon la revendication 5, dans lequel :
R¹ est choisi dans le groupe consistant en un atome d'hydrogène, un groupe hydroxy, -A, -O-A-, -C(O)-A et -SO₂-A ;
n est un nombre entier de 0 à 1 ;
chaque R² est indépendamment choisi dans le groupe consistant en un groupe carboxy, un atome d'halogène, -A, -C(O)-A, -C(O)O-A, un groupe cyano, -SO-A, -SO₂-A, -SO₂-NH₂, -SO₂-NH(alkyle (C₁-C₈) et -SO₂-N(alkyle (C₁-C₈))₂ ;
m est un nombre entier de 0 à 1 ;
chaque R³ est indépendamment choisi dans le groupe consistant en -A, -O-A, -S-A, -NH-A et -C(O)-A ;
p est un nombre entier de 1 à 2 ;
R⁴ est choisi dans le groupe consistant en un groupe hydroxy, -NH₂, -NH(A), -N(A)₂, -C(O)NH₂, -C(O)-NH(A), -SO₂-NH₂, -SO₂-NH(A) et -OC(O)-A, lorsque le R⁴ est dans une orientation β ;
R⁴ est choisi dans le groupe consistant en un groupe hydroxy, carboxy, cyano, -A, alcényle (C₂-C₈) alcényle (C₂-C₈)-A, alcynyle (C₂-C₈), alcynyle (C₂-C₈)-A, -O-A, -NH₂, -NH(A), -N(A)₂, -N(A)-C(O)-A, -NH-C(O)-A, -C(O)-N(A)₂, -C(O)-NH₂, -C(O)-NH-A, -SO₂-N(A)₂, -SO₂-NH(A), -SO₂-NH₂, -N(A)-SO₂-A, -NH-SO₂-A, -C(O)O-A, -OC(O)H et -OC(O)-A, lorsque le R⁴ est dans une orientation α ;
en variante, lorsque p est 2, deux groupes R⁴ peuvent être considérés ensemble comme étant oxo ou =N(OH) ;
q est un nombre entier de 0 à 1 ;
R⁵ est choisi dans le groupe consistant en un groupe carboxy, un atome d'halogène, un groupe alkyle (C₁-C₄) et -C(O)-A ; dans lequel le groupe alkyle est éventuellement substitué par un ou plusieurs substituants indépendamment choisis parmi un atome d'halogène, un groupe hydroxy, carboxy ou alcoxy (C₁-C₈) ;
dans lequel chaque A est indépendamment choisi dans le groupe consistant en un groupe alkyle (C₁-C₈), aryle, aralkyle, cycloalkyle (C₃-C₈), hétéroaryle et hétérocycloalkyle ; dans lequel le groupe aryle, aralkyle, cycloalkyle (C₃-C₈), hétéroaryle et hétérocycloalkyle est éventuellement substitué par un ou plusieurs substituants indépendamment choisis parmi un atome d'halogène, un groupe hydroxy, carboxy, alkyle (C₁-C₄), alcoxy (C₁-C₈), nitro, cyano, amino, alkylamino (C₁-C₄) ou di- (alkyle (C₁-C₄)) amino ;
ou sel pharmaceutiquement acceptable de celui-ci.

7. Composé selon la revendication 6, dans lequel :
R¹ est choisi dans le groupe consistant en un atome d' hydrogène et un groupe -SO₂-(alkyle (C₁-C₈)) ;
n est un nombre entier de 0 à 1 ;
R² est choisi dans le groupe consistant en un groupe -S- (alkyle (C₁-C₈)) ;
m est 0 ;
p est un nombre entier de 1 à 2 ;
R⁴ est choisi dans le groupe consistant en un groupe hydroxy, alcynyle (C₂-C₈)) et -O-C(O)-(alkyle (C₁-C₈) ;
en variante, lorsque p est 2, deux groupes R⁴ peuvent être considérés ensemble comme étant oxo ;
q est 0 ;
ou sel pharmaceutiquement acceptable de celui-ci.

8. Composé selon la revendication 7, dans lequel :
R¹ est choisi dans le groupe consistant en un atome d'hydrogène et un groupe -SO₂-CH₃ ;
n est un nombre entier de 0 à 1 ;
R² est -S-CH₃ ;
m est 0 ;
p est un nombre entier de 1 à 2 ;
R⁴ est choisi dans le groupe consistant en un groupe hydroxy, éthynyle et -OC(O)-n-butyle ;
en variante, lorsque p est 2, deux groupes R⁴ peuvent être considérés ensemble comme étant oxo ;
q est 0 ;
ou sel pharmaceutiquement acceptable de celui-ci.

9. Composition pharmaceutique comprenant un support pharmaceutiquement acceptable et un composé selon la revendication 1 ou la revendication 5.

10. Composition pharmaceutique préparée par mélange d'un composé selon la revendication 1 ou la revendication 5 et d'un support pharmaceutiquement acceptable.

11. Procédé de préparation d'une composition pharmaceutique comprenant le mélange d'un composé selon la revendication 1 ou la revendication 5 et d'un support pharmaceutiquement acceptable.

12. Composé selon la revendication 1 ou la revendication 5, pour une utilisation en thérapie.

13. Utilisation d'un composé selon la revendication 1 ou la revendication 5, pour la fabrication d'un médicament destiné au traitement d'un trouble médié par un récepteur d'oestrogènes.

14. Utilisation selon la revendication 13, dans laquelle le trouble médié par un récepteur d'oestrogènes est choisi dans le groupe consistant en des bouffées de chaleur, une sècheresse vaginale, une ostéopénie, une ostéoporose, une hyperlipidémie, une perte de la fonction cognitive, des syndromes cérébraux dégénératifs, des maladies cardiovasculaires, des maladies cérébrovasculaires, un cancer du tissu du sein, une hyperplasie du tissu du sein, un cancer de l'endomètre, une hyperplasie de l'endomètre, un cancer du col de l'utérus, une hyperplasie du col de l'utérus, un cancer de la prostate, une hypertrophie bénigne de la prostate, une endométriose, des fibromes utérins, une arthrose et une contraception.

15. Utilisation selon la revendication 14, dans laquelle le trouble médié par un récepteur d'oestrogènes est choisi dans le groupe consistant en l'ostéoporose, des bouffées de chaleur, une sècheresse vaginale, un cancer du sein et une endométriose.

16. Composition pharmaceutique selon la revendication 10, pour une utilisation en thérapie.

17. Utilisation d'une composition pharmaceutique selon la revendication 10, pour la fabrication d'un médicament destiné au traitement d'un trouble médié par un récepteur d'oestrogènes.
